# EUROPEAN PATENT APPLICATION

(11) **EP 4 585 687 A1**
(43) Date of publication of application: **16.07.2025**
(21) Application number: 23863257.4
(22) Date of filing: 07.09.2023
(51) Int. Cl.: C12N 15/13, A61K 39/395, A61P 31/14, C07K 16/10, C07K 16/46, C12N 1/15, C12N 1/19, C12N 1/21, C12N 5/10, C12N 15/63, C12P 21/08

(54) **HUMAN ANTIBODY AGAINST CORONAVIRUS VARIANTS OR ANTIGEN-BINDING FRAGMENT THEREOF**

(30) Priority: 08.09.2022 JP 2022142801
(71) Applicant: National University Corporation Kumamoto University, Kumamoto-shi, Kumamoto 860-8555 (JP); The University of Tokyo, Bunkyo-ku, Tokyo 113-8654 (JP)
(72) Inventor: MATSUSHITA Shuzo, Kumamoto-shi, Kumamoto 860-8555 (JP); KAKU Yu, Kumamoto-shi, Kumamoto 860-8555 (JP); KUWATA Takeo, kumamoto-shi, Kumamoto 860-8555 (JP); KAWAOKA Yoshihiro, Tokyo 113-8654 (JP); IMAI Masaki, Tokyo 113-8654 (JP); YAMAYOSHI Seiya, Tokyo 113-8654 (JP)
(74) Representative: Maiwald GmbH
(86) International application number: PCT/JP2023/032754
(87) International publication number: WO 2024/053719

(57) **Abstract**

It is an object of the present invention to provide an antibody against coronavirus (SARS-CoV-2) variants. It is also an object of the present invention to provide a pharmaceutical composition against coronavirus infection using the antibody. The present invention provides an antibody or antigen-binding fragment thereof that binds to the spike protein of the coronavirus and has the ability to neutralize coronaviruses, including the Omicron variant, and a pharmaceutical composition for the prevention or treatment of coronavirus infection, which contains the antibody or an antigen-binding fragment thereof.

## Description

### [Technical Field]

The present invention relates to a novel antibody or antigen-binding fragment thereof against variants of the causative virus (SARS-CoV-2) of coronavirus infection (COVID-19). The present invention also relates to therapeutic and preventive agents for coronaviruses using the antibody.

### [Background Art]

The novel coronavirus infection (COVID-19), first discovered in China in December 2019, has rapidly spread worldwide in a short period, has had a major impact on the global economy, and the ongoing uncertainty of the "with-corona" era has become a major social problem. In addition, the COVID-19 pandemic, which began in 2020, has entered a new phase with the spread of SARS-CoV-2 variants infection around the end of 2020.

The Omicron variant (B.1.1529, BA.1) of the novel coronavirus (SARS-CoV-2) was reported in November 2021 and rapidly became a globally dominant strain. BA.1 has more than 33 mutations in the spike protein and not only requires three doses of an mRNA vaccine but has also been reported to be resistant to existing neutralizing antibodies (Non-Patent Literature 1). Furthermore, sublineage variants (BA.1.1) and subvariants with different mutations (B.1.1.529.2, BA.2) have also been reported, and their containment has become a global challenge (Non-Patent Literature 2).

Neutralizing antibodies against SARS-CoV-2 were developed early in the pandemic, and their effectiveness in preventing severe illness when administered early had been demonstrated. In Japan, REGN-COV2 (Ronapreve^{®}, Regeneron) and Sotrovimab (Xevudy^{®}, GSK) were specially approved in 2021 and have been used in clinical settings. In addition to these, other antibodies such as LY-CoV555 (Eli Lilly) and AZD7442 (AstraZeneca) have also been developed. However, the Omicron variant has shown resistance to these antibodies, and even Sotrovimab, which was considered the only effective option, has been reported to be ineffective against the BA.2 subvariant (Non-Patent Literature 2).

On the other hand, since January 2022, numerous reports have been published regarding neutralizing antibodies against the Omicron variant. One such antibody, S2K146, has been reported to exhibit a binding pattern similar to ACE-2 (Non-Patent Literature 3). Another antibodies S2X259 and S2H97 classified as Class 3, similar to Sotrovimab which react with regions outside the receptor-binding motif (RBM), have also been reported (Non-Patent Literature 4). However, many Class 3 antibodies exhibit weak neutralizing activity and have drawbacks, such as being ineffective against the BA.2 subvariant. On the other hand, LY-CoV1404 (Bebtelovimab), developed by Eli Lilly, has demonstrated exceptionally strong activity (Non-Patent Literature 2, Non-Patent Literature 5), resulting in that FDA granted its emergency use authorization (EUA) in February 2022. Furthermore, a research group in China reported that they isolated antibodies capable of strongly neutralizing the Omicron variant from volunteers who had received three doses of an inactivated vaccine (Non-Patent Literature 1).

Furthermore, in 2020, the present inventors discovered and reported an antibody (9-105 antibody) that exhibits strong neutralizing activity against both the wild-type SARS-CoV-2 and its variants (B.1.1.7 strain, mink cluster 5 strain, B.1.351 strain, P.1 strain, B.1.617.1 strain, and B.1.617.2 strain) (Patent Literature 1, Non-Patent Literature 6). The 9-105 antibody has been found to be effective at low concentrations against existing viral variants. However, the present inventors confirmed that its effectiveness is lower against the Omicron variant compared to those strains.

Thus, the development of antibodies against SARS-CoV-2 variants and therapies utilizing them remains a top global priority. There is a continuing need for the development of antibodies capable of neutralizing SARS-CoV-2 variants, including the Omicron variant.

### [Citation List]

### [Patent Document]

Patent Document 1: WO2022/044573

### [Non-Patent Document]

Non-Patent Document 1: Wang K. et al., Vol.603, pp.919-925, Nature. 2022
Non-Patent Document 2: Iketani S. et al., Vol.694, pp.553-556, Nature. 2022
Non-Patent Document 3: Park YJ et al., Vol.375, pp.449-454, Science, 2022
Non-Patent Document 4: Cameroni E. et al., Vo.602, pp.664-670, Nature. 2022
Non-Patent Document 5: Westendorf et al., bioRxiv, 2022, doi: 10.1101/2021.04.30.442182
Non-Patent Document 6: Kaku Y. et al., Cell Report Vol 36, 2, 109385, Julyu 12, 2021: https://doi.org/10.1016/j.celrep.2021.109385.

### [Summary of the Invention]

### [Technical Problem]

It is an object of the present invention to provide an antibody or antigen-binding fragment thereof that exhibits neutralizing activity against variants of the coronavirus (SARS-CoV-2), particularly the Omicron variant. The present invention also provides a pharmaceutical composition against coronavirus infection using the antibody or antigen-binding fragment thereof.

### [Solution to Problem]

The inventors conducted intensive research to develop a treatment for coronavirus infections using neutralizing antibodies against SARS-CoV-2 variants, particularly the Omicron variant, with a specific focus on therapies for preventing severe disease. As a result, a case was identified in which an individual, after receiving a SARS-CoV-2 vaccine, was infected with the Delta variant and subsequently developed potent cross-neutralizing antibodies. By screening peripheral blood B cells from this case using two types of spike proteins, an antibody capable of neutralizing the Omicron variant was developed, leading to the completion of the present invention. The present invention includes the following aspects.
[1] An antibody or antigen binding fragment thereof that binds to the spike protein of the SARS-CoV-2 and has the ability to neutralize the SARS-CoV-2 virus (particularly the Omicron variant), comprising the following heavy chains CDR1-3 and light chains CDR1-3:
   (1) a heavy chain CDR1 selected from the group consisting of:
      a heavy chain CDR1 represented by SEQ ID NO: 9 (GYSFTASY),
      a heavy chain CDR1 represented by SEQ ID NO: 11 (GYIFTNYW),
      a heavy chain CDR1 represented by SEQ ID NO: 13 (GFTFRSHA),
      a heavy chain CDR1 represented by SEQ ID NO: 15 (GIIVSRNY),
      a heavy chain CDR1 consisting of a sequence in which one amino acid is deleted, substituted or added in any of heavy chains CDR1 selected from the group consisting of the sequences represented by SEQ ID NOs: 9, 11, 13 and 15, and
      a heavy chain CDR1 having 85% or more substantial identity to any of heavy chains CDR1 selected from the group consisting of the sequences represented by SEQ ID NOs: 9, 11, 13 and 15,
   (2) a heavy chain CDR2 selected from the group consisting of:
      a heavy chain CDR2 represented by SEQ ID NO: 17 (INPGDDNT),
      a heavy chain CDR2 represented by SEQ ID NO: 18 (INPRDSDT),
      a heavy chain CDR2 represented by SEQ ID NO: 19 (IWYDGSNK),
      a heavy chain CDR2 represented by SEQ ID NO: 20 (IYSGGTT),
      a heavy chain CDR2 consisting of a sequence in which one amino acid is deleted, substituted or added in any of heavy chains CDR2 selected from the group consisting of the sequences represented by SEQ ID NOs: 17, 18, 19 and 20, and
      a heavy chain CDR2 having 85% or more substantial identity to any of heavy chains CDR2 selected from the group consisting of the sequences represented by SEQ ID NOs: 17, 18, 19 and 20,
   (3) a heavy chain CDR3 selected from the group consisting of:
      a heavy chain CDR3 represented by SEQ ID NO: 21 (TRSTRDYVWGNYRYTPGYYLDY),
      a heavy chain CDR3 represented by SEQ ID NO: 23 (ARQQGGFGDLGAYNWFDP),
      a heavy chain CDR3 represented by SEQ ID NO: 25 (AREGIAVPGNGADAFDI),
      a heavy chain CDR3 represented by SEQ ID NO: 27 (ARDPPHRRGSY),
      a heavy chain CDR3 consisting of a sequence in which one or two amino acids are deleted, substituted or added in any of heavy chains CDR3 selected from the group consisting of the sequences represented by SEQ ID NOs: 21, 23, 25 and 27, and a heavy chain CDR3 having 90% or more substantial identity
      to any of heavy chains CDR3 selected from the group consisting of the sequences represented by SEQ ID NOs: 21, 23, 25 and 27,
   (4) a light chain CDR1 selected from the group consisting of:
      a light chain CDR1 represented by SEQ ID NO: 10 (QTISNY),
      a light chain CDR1 represented by SEQ ID NO: 12 (SSNIGSNT),
      a light chain CDR1 represented by SEQ ID NO: 14 (QSIGNF),
      a light chain CDR1 represented by SEQ ID NO: 16 (QDINKY),
      a light chain CDR1 consisting of a sequence in which one amino acid is deleted, substituted or added in any of light chains CDR1 selected from the group consisting of the sequences represented by SEQ ID NOs: 10, 12, 14 and 16, and
      a light chain CDR1 having 80% or more substantial identity to any of light chains CDR1 selected from the group consisting of the sequences represented by SEQ ID NOs: 10, 12, 14 and 16,
   (5) a light chain CDR2 selected from the group consisting of:
      a light chain CDR2 represented by the sequence A (AAS),
      a light chain CDR2 represented by the sequence B (RDH), and
      a light chain CDR2 represented by the sequence C (DAS), and
   (6) a light chain CDR3 selected from the group consisting of:
      a light chain CDR3 represented by SEQ ID NO: 22(QQGYITPIT),
      a light chain CDR3 represented by SEQ ID NO: 24(AAWDDSLNGWV),
      a light chain CDR3 represented by SEQ ID NO: 26(QQTYSTPYT),
      a light chain CDR3 represented by SEQ ID NO: 28(QQSDNLPPT),
      a light chain CDR3 consisting of a sequence in which one
      amino acid is deleted, substituted or added in any of light chains CDR3 selected from the group consisting of the sequences represented by SEQ ID NOs: 22, 24, 26 and 28, and a light chain CDR3 having 85% or more substantial identity with any of light chains CDR3 selected from the group consisting of the sequences represented by SEQ ID NOs: 22, 24, 26 and 28.
[2] The antibody or antigen-binding fragment thereof described in [1], wherein the heavy chain CDR1 is a heavy chain CDR1 selected from the group consisting of the sequences represented by SEQ ID NOs: 9, 11, 13 and 15, the heavy chain CDR2 is a heavy chain CDR2 selected from the group consisting of the sequences represented by SEQ ID NOs: 17, 18, 19 and 20, the heavy chain CDR3 is a heavy chain CDR3 selected from the group consisting of the sequences represented by SEQ ID NOs: 21, 23, 25 and 27, the light chain CDR1 is a light chain CDR1 selected from the group consisting of the sequences represented by SEQ ID NOs: 10, 12, 14 and 16, the light chain CDR2 is a light chain CDR2 selected from the group consisting of the sequences A, B and C, and the light chain CDR3 is a light chain CDR3 selected from the group consisting of the sequences represented by SEQ ID NOs: 22, 24, 26 and 28.
[3] The antibody or antigen-binding fragment thereof described in [1], wherein the combination of heavy chain CDR1, heavy chain CDR2, heavy chain CDR3, light chain CDR1, light chain CDR2, and light chain CDR3 is selected from the group consisting of:
   (1) the heavy chain CDR1 represented by SEQ ID NO: 9, the heavy chain CDR2 represented by SEQ ID NO: 17, the heavy chain CDR3 represented by SEQ ID NO: 21, the light chain CDR1 represented by SEQ ID NO: 10, the light chain CDR2 represented by the sequence A, and the light chain CDR3 represented by SEQ ID NO: 22.
   (2) the heavy chain CDR1 represented by SEQ ID NO: 11, the heavy chain CDR2 represented by SEQ ID NO: 18, the heavy chain CDR3 represented by SEQ ID NO: 23, the light chain CDR1 represented by SEQ ID NO: 12, the light chain CDR2 represented by the sequence B, and the light chain CDR3 represented by SEQ ID NO: 24.
   (3) the heavy chain CDR1 represented by SEQ ID NO: 13, the heavy chain CDR2 represented by SEQ ID NO: 19, the heavy chain CDR3 represented by SEQ ID NO: 25, the light chain CDR1 represented by SEQ ID NO: 14, the light chain CDR2 represented by the sequence A, and the light chain CDR3 represented by SEQ ID NO: 26.
   (4) the heavy chain CDR1 represented by SEQ ID NO: 15, the heavy chain CDR2 represented by SEQ ID NO: 20, the heavy chain CDR3 represented by SEQ ID NO: 27, the light chain CDR1 represented by SEQ ID NO: 16, the light chain CDR2 represented by the sequence C, and the light chain CDR3 represented by SEQ ID NO: 28.
[4] The antibody or antigen-binding fragment thereof described in [1], comprising the heavy chain variable region represented by SEQ ID NO: 1 and the light chain variable region represented by SEQ ID NO: 2.
[5] The antibody or antigen-binding fragment thereof described in [1], comprising the heavy chain variable region represented by SEQ ID NO: 3 and the light chain variable region represented by SEQ ID NO: 4.
[6] The antibody or antigen-binding fragment thereof described in [1], comprising the heavy chain variable region represented by SEQ ID NO: 5 and the light chain variable region represented by SEQ ID NO: 6.
[7] The antibody or antigen-binding fragment thereof described in [1], comprising the heavy chain variable region represented by SEQ ID NO: 7 and the light chain variable region represented by SEQ ID NO: 8.
[8] The antibody or antigen-binding fragment thereof described in any one of [1] to [7], wherein in a neutralization assay using pseudoviruses, the antibody activity in neutralizing the BA.1 strain of the SARS-CoV-2 Omicron variants has a neutralizing ability expressed as 50% inhibitory concentration (IC₅₀ µg/mL) within the range of about 1 µg/mL or less (preferably about 0.8 µg/mL or less, more preferably about 0.5 µg/mL or less).
[9] The antibody or antigen-binding fragment thereof described in any one of [1] to [7], having neutralizing activity against at least four (preferably at least five) variants selected from the group consisting of the SARS-CoV-2 Omicron variants BA.1, BA.1.1, BA.2, BA.2.12.1, BA.3, BA.4/5, BQ.1.1, CH.1.1, and XBB.1.5, as expressed as 50% inhibitory concentration (IC₅₀µg/mL) within the range of about 1 µg/mL or less (preferably about 0.8 µg/mL or less, more preferably about 0.5 µg/mL or less, and even more preferably about 0.2 µg/mL or less).
[10] The antibody or antigen-binding fragment thereof described in any one of [1] to [9], selected from the group consisting of immunoglobulin molecules, monoclonal antibodies, human antibodies, chimeric antibodies, CDR-grafted antibodies, Fab, Fab', F(ab')2, Fd, Fv, disulfide-bound Fv, scFv, single domain antibodies, nanobody antibodies, diabody antibodies, bispecific antibodies, and multi-specific antibodies.
[11] A nucleic acid encoding the antibody or antigen-binding fragment thereof described in any one of [1]-[10].
[12] A vector comprising the nucleic acid described in [11].
[13] A host cell comprising the nucleic acid described in [11] or the vector described in [12].
[14] A method for producing the antibody or antigen-binding fragment thereof described in any one of [1] to [10], comprising a step of culturing the host cell described in [13] under conditions suitable for expressing the antibody or antigen-binding fragment thereof.
[15] A pharmaceutical composition comprising the antibody or antigen-binding fragment thereof described in any one of [1] to [10] and a pharmaceutically acceptable carrier.
[16] The pharmaceutical composition described in [15], further comprising at least one additional neutralizing antibody or its antigen-binding fragment thereof against SARS-CoV-2 and/or SARS-CoV-2 variants.
[17] The pharmaceutical composition described in [16], wherein the additional neutralizing antibody or its antigen-binding fragment thereof is an antibody or its antigen-binding fragment thereof comprising the heavy chain CDR1 represented by SEQ ID NO: 33 (GITVSSNY), the heavy chain CDR2 represented by SEQ ID NO: 37 (IYSGGST), the heavy chain CDR3 represented by SEQ ID NO: 38 (ARDLEAAGGMDV), the light chain CDR1 represented by SEQ ID NO: 34 (QSVPSIY), the light chain CDR2 represented by the sequence D (GAS) and the light chain CDR3 represented by SEQ ID NO: 39 (QQYGSSPGT), or an antibody or its antigen-binding fragment thereof comprising the heavy chain CDR1 represented by SEQ ID NO: 35 (GLTVSRNY), the heavy chain CDR2 represented by SEQ ID NO: 37 (IYSGGST), the heavy chain CDR3 represented by SEQ ID NO: 40 (ARPIVGARAGMDV), the light chain CDR1 represented by SEQ ID NO: 36 (QDISNY), the light chain CDR2 represented by the sequence E (DAS) and the light chain CDR3 represented by SEQ ID NO: 41 (QQYDNLPGT).
[18] The pharmaceutical composition described in [16], wherein the additional neutralizing antibody or its antigen-binding fragment thereof is an antibody or its antigen-binding fragment thereof comprising the heavy chain variable region represented by SEQ ID NO: 29 and the light chain variable region represented by SEQ ID NO: 30, or an antibody or its antigen-binding fragment thereof comprising the heavy chain variable region represented by SEQ ID NO: 31 and the light chain variable region represented by SEQ ID NO: 32.
[19] A pharmaceutical composition for the prevention or treatment of SARS-CoV-2 virus infection (preferably SARS-CoV-2 variant virus infection, and more preferably infection by the Omicron variant of SARS-CoV-2), comprising the antibody or antigen-binding fragment thereof described in any one of [1] to [10].
[20] The pharmaceutical composition described in [19], further comprising at least one additional neutralizing antibody or its antigen-binding fragment thereof against SARS-CoV-2 and/or SARS-CoV-2 variants.
[21] The pharmaceutical composition described in [20], wherein the additional neutralizing antibody or its antigen-binding fragment thereof is an antibody or its antigen-binding fragment thereof comprising the heavy chain CDR1 represented by SEQ ID NO: 33 (GITVSSNY), the heavy chain CDR2 represented by SEQ ID NO: 37 (IYSGGST), the heavy chain CDR3 represented by SEQ ID NO: 38 (ARDLEAAGGMDV), the light chain CDR1 represented by SEQ ID NO: 34 (QSVPSIY), the light chain CDR2 represented by the sequence D (GAS) and the light chain CDR3 represented by SEQ ID NO: 39 (QQYGSSPGT), or an antibody or its antigen-binding fragment thereof comprising the heavy chain CDR1 represented by SEQ ID NO: 35 (GLTVSRNY), the heavy chain CDR2 represented by SEQ ID NO: 37 (IYSGGST), the heavy chain CDR3 represented by SEQ ID NO: 40 (ARPIVGARAGMDV), the light chain CDR1 represented by SEQ ID NO: 36 (QDISNY), the light chain CDR2 represented by the sequence E (DAS) and the light chain CDR3 represented by SEQ ID NO: 41 (QQYDNLPGT).
[22] The pharmaceutical composition described in [20], wherein the additional neutralizing antibody or its antigen-binding fragment thereof is an antibody or its antigen-binding fragment thereof comprising the heavy chain variable region represented by SEQ ID NO: 29 and the light chain variable region represented by SEQ ID NO: 30, or an antibody or its antigen-binding fragment thereof comprising an antibody or its antigen-binding fragment thereof comprising the heavy chain variable region represented by SEQ ID NO: 31 and the light chain variable region represented by SEQ ID NO: 32.
[23] The pharmaceutical composition described in any one of [15] to [22], wherein it is administered in combination with another anti-coronaviral drug.
[24] The pharmaceutical composition described in [23], wherein the anti-coronaviral drug is at least one anti-coronaviral drug selected from remdesivir, favipiravir, dexamethasone, ciclenisonide, nafamostat, camostat, ivermectin, bamilanibimab, etesevimab, casilibimab, imdevimab, and HIV protease inhibitors (for example, Lopinavir, ritonavir or their combination, nelfinavir).
[25] Use of the antibody or its antigen-binding fragment thereof described in any one of [1] to [10] in the manufacture of a pharmaceutical composition for the prevention or treatment of SARS-CoV-2 virus infection (preferably SARS-CoV-2 variant virus infection, and more preferably infection by the Omicron variant of SARS-CoV-2).

### [Advantageous Effect of the Invention]

The present invention provides a novel antibody neutralizing SARS-CoV-2 variants, particularly the Omicron variant.

### [Brief Description of Drawings]

[Fig.1] Figure 1 shows the amino acid sequences of the heavy chain variable regions consisitng of the framework regions and CDR1-3 regions and the light chain variable regions consisting of the framework regions and CDR1-3 regions of four antibodies of the present invention (Clones 1-58, 3-1, 1-44, and 4-66).
[Fig.2] Figure 2 shows the amino acid sequences of the heavy chain variable regions consisitng of the framework regions and CDR1-3 regions and the light chain variable regions consisitng of the framework regions and CDR1-3 regiond of neutralizing antibodies against SARS-CoV-2 variants (9-105 and 10-121 antibodies) previously reported by the inventors.

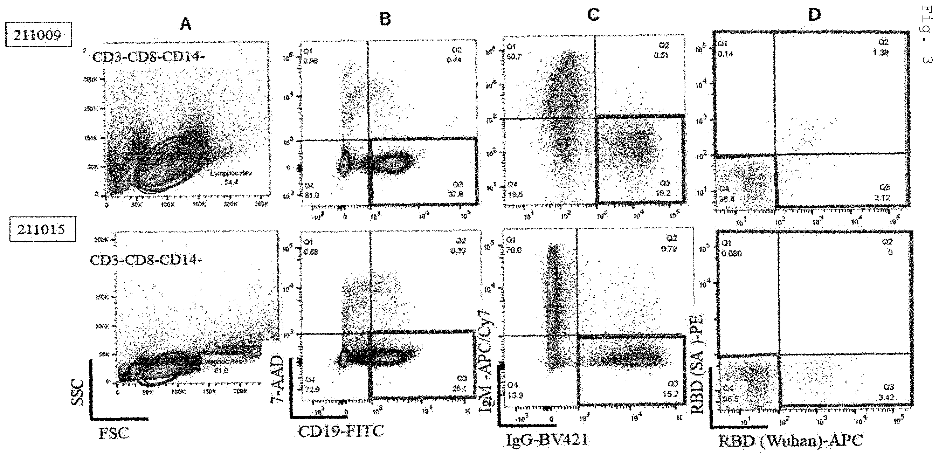

[Fig.3] Figure 3 presents the results of flow cytometry analysis using peripheral blood mononuclear cells isolated from a blood sample of a recovered case infected with the SARS-CoV-2 Delta variant after vaccination (211009 and 211015 represent the results of sorting conducted on two different dates). The results include: A: Sorting based on forward scatter (FSC), which reflects cell size, and side scatter (SSC), which provides information on cell morphology and internal structure; B: Sorting based on CD19 and 7-ADD markers; C: Sorting based on IgG and IgM markers; and D: Sorting based on RBD (Wuhan) and RBD (SA) markers. The thick red lines indicate the gating ranges used for sorting.
[Fig.4] Figure 4 shows the results of screening for antibodies that neutralize the Omicron variant (BA.1 strain). The left and right bars for each clone represent results from two independent experiments.
[Fig.5] Figure 5 presents the results of a neutralization assay using the antibodies of the present invention (1-58, 3-1, 1-44, and 4-66) against the Omicron variant (BA.1).
[Fig. 6] Figure 6 shows the results of neutralization assays using the antibodies of the present invention (1-58, 3-1, 1-44, and 4-66) against various SARS-CoV-2 variants.
[Fig.7] Figure 7 presents the results of an RBD-ACE2 binding inhibition assay using the antibodies of the present invention (1-58, 3-1, 1-44, and 4-66).
[Fig.8] Figure 8 shows the comparison results of the neutralizing activity (IC₅₀) of the antibodies of the present invention (1-58, 3-1, 1-44, and 4-66) with those of existing antibodies against various SARS-CoV-2 variants.
[Fig.9] Figure 9 shows the comparison results of the neutralizing activity (IC₉₀) of the antibodies of the present invention (1-58, 3-1, 1-44, and 4-66) with those of existing antibodies against various SARS-CoV-2 variants.
[Fig.10] Figure 10 presents the results of neutralization assays using the antibodies of the present invention (1-58 and 4-66) against different subvariants of the SARS-CoV-2 Omicron variant.
[Fig.11] Figure 11 shows the results confirming the therapeutic efficacy of the antibodies of the present invention (1-58 and 4-66) in animals infected with the Omicron subvariant (XBB.1.5). The upper figure illustrates an overview of the study design. The lower figure shows the result of measuring the viral load in the lungs and serum after antibody administration.

### [Description of Embodiments]

Hereinafter, the present invention will be illustrated in more detail along with preferred methods and materials which can be used in practice of the present invention. However, the present invention is not limited to the following embodiments. Unless otherwise specified in the sentences, any technical terms and scientific terms used in the present specification have the same meaning as those generally understood by those of ordinary skill in the art to which the present invention belongs. Any materials and methods equivalent or similar to those described in the present specification can be used for practicing the present invention. All publications and patents cited herein in connection with the present invention described herein are incorporated by reference, for example, as indicating methodology, materials, etc. that can be used in the present invention.

In the present specification, the description of "A to B" indicating a numerical range means a numerical range including A and B as endpoints. The same applies to "A through B". Moreover, in the present specification, the term "about" is used in the sense of allowing ±10%. As used herein, "one or more" means "one, two, three, four or more" unless the meaning is explicitly indicated or clear from the surrounding context.

"SARS-CoV-2", also called "novel coronavirus", refers to a newly emerging coronavirus first isolated in Wuhan, China in 2020 and identified as the cause of an outbreak of severe acute respiratory illness. It binds to the human host cell receptor angiotensin-converting enzyme 2 (ACE2) via the viral spike protein. Various SARS-CoV-2 variants classified in the SARS coronavirus group have been isolated and identified. SARS-CoV-2 has undergone repeated mutations from the original Wuhan strain, resulting in that new variants such as prototype (614G), Alpha strain (B.1.1.7), Beta strain (B.1.351), Gamma strain (P.1), Delta strain (B.1.617.2), Kappa strain (B.1.617.1), Lambda strain (C.37), and Mu strain (B.1.621) have been reported successively and have caused widespread outbreaks. More recently, highly transmissible Omicron (BA.1, BA.1.1, BA.2, BA.2.12.1, BA.3, BA.4/5) variants have been reported and have become a global concern. Furthermore, sublineages of the Omicron variant (BQ.1.1, CH.1.1, and XBB.1.5) continue to be reported. There is also the possibility of undetected variants or the emergence of new variants in the future. Herein, the terms "SARS-CoV-2," "SARS-CoV-2 virus," "SARS-CoV-2 variant," or "SARS-CoV-2 viral variant" are not limited to the variants listed above but also include any other SARS-CoV-2 variants that may be detected in the future. Additionally, herein when referring to "SARS-CoV-2 spike protein," unless explicitly stated otherwise or clearly indicated by the context, the term is not limited to the spike proteins of the above-mentioned SARS-CoV-2 variants but includes spike proteins of any SARS-CoV-2 variants that may be newly detected in the future.

"SARS-CoV-2 spike protein", "spike protein" or "SARS-CoV-2(S)" means the spike protein of SARS-CoV-2 coronavirus. The SARS-CoV-2 spike protein is a 1273 amino acid type I membrane glycoprotein that forms a trimer and exists as a spike (peplomer) protruding from the surface of the SARS-CoV-2 virus particle. The SARS-CoV-2 spike protein has host receptor binding and membrane fusion functions and binds to ACE2 through an approximately 215 amino acid long receptor binding domain (RBD) present in the S1 subunit. The amino acid sequence of full-length SARS-CoV-2 spike protein (SEQ ID NO: 42) has been deposited in GeneBank under accession number YP_009724390. In the present specification, the SARS-CoV-2 spike protein includes recombinant SARS-CoV-2 spike proteins or fragments thereof.

"ACE2" is angiotensin converting enzyme 2, the receptor to which SARS-CoV-2 binds. ACE2 is an 805 amino acid type I transmembrane glycoprotein present on the cell surface that segregates angiotensin II into angiotensin 1-7 polypeptides. The isolated polypeptide has effects such as cardioprotection, vasodilation, and the like. ACE2 used herein means human ACE2 unless otherwise specified. ACE2 acts as a functional receptor for the SARS-CoV-2 virus. Viruses bind ACE2 through their RBD. It has been reported that TMPRSS2, a serine transmembrane protease present on the host cell membrane, is required for binding of ACE2 to a virus and intracellular entry via membrane fusion.

Aspects of the present invention are described below, but the present invention is not limited thereto.

One aspect of the present invention is an antibody or antigen-binding fragment thereof having a specific amino acid sequence that binds to the SARS-CoV-2 spike protein and is useful for binding to the SARS-CoV-2 spike protein to neutralize SARS-CoV-2 variants, particularly the Omicron variant. Hereinafter, in the present specification, the antibody and antigen-binding fragment thereof that bind to the SARS-CoV-2 spike protein are collectively referred to as "antibodies against SARS-CoV-2", "anti-SARS-CoV-2 antibodies", "antibodies against the SARS-CoV-2 spike protein", "anti-SARS-CoV-2(S) antibodies", or simply as "antibodies" or "antibodies" of the present invention, but if the text clearly refers to either an antibody or an antigen-binding fragment thereof, or if it can be clearly understood from the surrounding context that it is one of them, they are understood to mean either antibodies that bind the spike protein of SARS-CoV-2 or fragments that bind the spike protein of SARS-CoV-2.

As used herein, the term "antibody" is used in the sense of including complete antibodies and fragments thereof, but when the sentence clearly indicates either a complete antibody or an antibody fragment, or from the context before and after, where either of these can be clearly understood, it is understood to mean either an antibody or a fragment of an antibody. It is clear to those skilled in the art that antigen-binding fragments (fragments of antibodies that bind to antigens) can compete with intact antibodies for specific binding to antigens and exhibit similar effects of antibodies. See Fundamental Immunology, Chapter 7 (Paul, W., ed., 2nd ed., Raven Press, NY (1989)).

In certain embodiments, the antibodies of the present invention are useful for inhibiting the binding of the virus to its host cell receptor, angiotensin-converting enzyme 2 (ACE2), and preventing entry of the SARS coronavirus into host cells. In certain embodiments, the antibodies of the present invention exhibit antiviral activity by inhibiting SARS-CoV-2 spike protein-mediated cell fusion. In certain other embodiments, the antibodies of the present invention prevent, treat, or ameliorate at least one symptom of SARS-CoV-2 virus infection in a subject (preferably a human). In certain other embodiments, the antibodies of the present invention are prophylactically or therapeutically administered to a subject (preferably a human) infected with or at risk of being infected with the SARS-CoV-2 virus.

The antibodies of the present invention can be full-length (e.g., IgG1, IgG2 or IgG4), or can include an antigen-binding portion thereof (e.g., Fab, Fab', F(ab')2, Fd, Fv, disulfide Fv, scFv, single domains, nanobodies, diabodies), which may be modified to improve functionality or to remove nonessential effector functions. Also, in certain embodiments, the antibodies of the present invention can be bispecific or multispecific.

In one aspect, the present invention provides an isolated recombinant monoclonal antibody or antigen-binding fragment thereof that specifically binds to the spike protein of SARS-CoV-2. In certain embodiments, the antibodies of the present invention is a fully human monoclonal antibody. In one aspect, the antibodies of the present invention bind to an epitope within the receptor binding domain (RBD) of the spike protein of SARS-CoV-2. In certain embodiments, the antibodies of the present invention bind to one or a plurality of amino acids selected from amino acids 322-536 (corresponding to the RBD sequences) of the SARS-CoV-2 spike protein (registered in GenBank as accession number YP_009724390) (SEQ ID NO: 42). In addition, the whole genome information of SARS-CoV-2 virus is registered in GeneBank as Genome Reference Sequence: NC_045512. In certain embodiments, the antibodies of the present invention bind to the spike protein of various SARS-CoV-2 variants, preferably the Omicron variant, and can also be used against these variants.

Representative anti-SARS-CoV-2 antibodies of the present invention include, for example, antibodies having the heavy chain variable region represented by the amino acid sequences described in SEQ ID NO: 1, 3, 5, or 7. Representative anti-SARS-CoV-2 antibodies of the present invention include, for example, antibodies having the light chain variable region represented by the amino acid sequences described in SEQ ID NO: 2, 4, 6, or 8. More representative anti-SARS-CoV-2 antibodies of the present invention include, for example, antibodies that have combinations of SEQ ID NO: 1 and SEQ ID NO: 2, SEQ ID NO: 3 and SEQ ID NO: 4, SEQ ID NO: 5 and SEQ ID NO: 6, or SEQ ID NO: 7 and SEQ ID NO: 8 as a set of amino acid sequences of the heavy chain variable region and the light chain variable region.

The amino acid sequences of the heavy chain variable region (HCVR), the light chain variable region (LCVR), the heavy chain complementarity determining regions (HCDR1, HCDR2, and HCDR3), and the light chain complementarity determining regions (LCDR1, LCDR2 and LCDR3) of four representative anti-SARS-CoV-2 antibodies of the present invention are listed in the table below. The sequence A represents the amino acid sequence AAS, the sequence B represents the amino acid sequence RDH, and the sequence C represents the amino acid sequence DAS.

**Table 1**

| 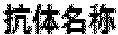 | HCVR | HCDR1 | HCDR2 | HCDR3 | LCVR | LCDR1 | LCDR2 | LCDR3 |
|---|---|---|---|---|---|---|---|---|
| 1-58 | 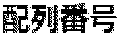 1 | 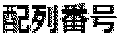 9 | 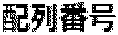 17 | 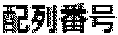 21 | 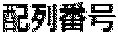 2 | 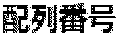 10 | 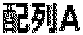 A | 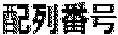 22 |
| 3-1 | 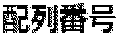 3 | 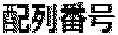 11 | 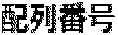 18 | 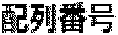 23 | 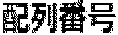 4 | 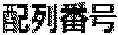 12 | 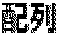 B | 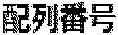 24 |
| 1-44 | 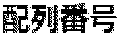 5 | 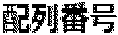 13 | 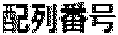 19 | 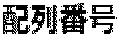 25 | 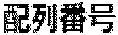 6 | 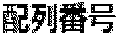 14 | 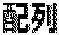 A | 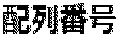 26 |
| 4-66 | 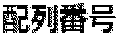 7 | 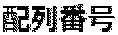 15 | 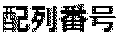 20 | 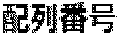 27 | 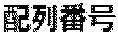 8 | 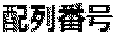 16 | 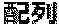 C | 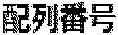 28 |

The present invention provides an antibody or antigen-binding fragment thereof containing HCVR containing any of HCVR amino acid sequences listed in Table 1, or an amino acid sequence selected from sequences having at least 70%, 75% or 80% substantial identity, preferably at least 90%, 92% or 95% substantial identity, and more preferably at least 98% or 99% substantial identity thereto.

In addition, the present invention provides an antibody or antigen-binding fragment thereof containing LCVR containing any of LCVR amino acid sequences listed in Table 1, or an amino acid sequence selected from sequences having at least 70%, 75% or 80% substantial identity, preferably at least 90%, 92% or 95% substantial identity, and more preferably at least 98% or 99% substantial identity thereto.

In addition, the present invention provides an antibody or antigen-binding fragment thereof containing a HCVR and LCVR amino acid sequence pair (HCVR/LCVR), containing any of HCVR amino acid sequences listed in Table 1 and any of LCVR amino acid sequences listed in Table 1, preferably, containing combinations of SEQ ID NO: 1 and SEQ ID NO: 2, SEQ ID NO: 3 and SEQ ID NO: 4, SEQ ID NO: 5 and SEQ ID NO: 6, or SEQ ID NO: 7 and SEQ ID NO: 8, which form a pair.

The present invention also provides an antibody or antigen-binding fragment thereof containing a heavy chain CDR1 (HCDR1) that includes an amino acid sequence selected from any of the four HCDR1 amino acid sequences listed in Table 1, or an amino acid sequence having at least 85%, preferably at least 90% substantial identity thereto.

In addition, the present invention provides an antibody or antigen-binding fragment thereof containing a heavy chain CDR2 (HCDR2) that includes an amino acid sequence selected from any of the two HCDR2 amino acid sequences listed in Table 1, or an amino acid sequence having at least 85%, preferably at least 90% substantial identity thereto.

In addition, the present invention provides an antibody or antigen-binding fragment thereof containing a heavy chain CDR3 (HCDR3) that includes an amino acid sequence selected from any of the four HCDR3 amino acid sequences listed in Table 1, or an amino acid sequence having at least 90%, preferably at least 95% substantial identity thereto.

In addition, the present invention provides an antibody or antigen-binding fragment thereof containing a light chain CDR1 (LCDR1) that includes an amino acid sequence selected from any of the four LCDR1 amino acid sequences listed in Table 1, or an amino acid sequence having at least 80%, preferably at least 90% substantial identity thereto.

In addition, the present invention provides an antibody or antigen-binding fragment thereof containing a light chain CDR2 (LCDR2) that includes an amino acid sequence selected from any of the three LCDR2 amino acid sequences listed in Table 1.

In addition, the present invention provides an antibody or antigen-binding fragment thereof containing a light chain CDR3 (LCDR3) that includes an amino acid sequence selected from any of the four LCDR3 amino acid sequences listed in Table 1, or an amino acid sequence having at least 85%, preferably at least 90% substantial identity thereto.

In addition, the present invention provides an antibody or antigen-binding fragment thereof containing a HCDR3 and LCDR3 amino acid sequence pair (HCDR3/LCDR3), containing any of four HCDR3 amino acid sequences listed in Table 1 and any of four LCDR3 amino acid sequences listed in Table 1, which form a pair. According to certain embodiments, the present invention provides an antibody or antigen-binding fragment thereof containing an HCDR3/LCDR3 amino acid sequence pair contained in any of four representative anti-SARS-CoV-2 antibodies listed in Table 1. In certain embodiments, the HCDR3/LCDR3 amino acid sequence pair is selected from the group consisting of SEQ ID NOs: 21 and 22 (1-58), 23 and 24 (3-1), 25 and 26 (1-44), and 18 and 29 (4-66).

In addition, the present invention provides an antibody or antigen-binding fragment thereof containing a set of six CDRs (i.e., a set of HCDR1-HCDR2-HCDR3+LCDR1-LCDR2-LCDR3) contained in any of representative anti-SARS-CoV-2 antibodies listed in Table 1. In certain embodiments, the HCDR1-HCDR2-HCDR3+LCDR1-LCDR2-LCDR3 amino acid sequence set is selected from the group consisting of SEQ ID NO:9-SEQ ID NO:17-SEQ ID NO:21 + SEQ ID NO:10-Sequence A-SEQ ID NO:22 (1-58), SEQ ID NO:11-SEQ ID NO:18-SEQ ID NO:23 + SEQ ID NO:12-Sequence B-SEQ ID NO:24 (3-1), SEQ ID NO:13-SEQ ID NO:19-SEQ ID NO:25 + SEQ ID NO:14-Sequence A-SEQ ID NO:26 (1-44), and SEQ ID NO:15-SEQ ID NO:20-SEQ ID NO:27 + SEQ ID NO:16-Sequence C-SEQ ID NO:28 (4-66).

In another embodiment, the present invention provides an antibody or antigen-binding fragment thereof containing a combination of any of a set of three CDRs contained in the HCVR amino acid sequence (i.e., HCDR1-HCDR2-HCDR3) and any of a set of three CDRs contained in the LCVR amino acid sequence (i.e., the set of LCDR1-LCDR2-LCDR3) defined by any of representative anti-SARS-CoV-2 antibodies listed in Table 1. In certain embodiments, the set of HCDR1-HCDR2-HCDR3 is selected from the group consisting of sets of SEQ ID NOs: 9-17-21, SEQ ID NOs: 11-18-23, SEQ ID NOs: 13-19-25 and SEQ ID NOs: 15-20-27; and the set of LCDR1-LCDR2-LCDR3 is selected from the group consisting of sets of SEQ ID NO: 10-Sequence A-SEQ ID NO:22, SEQ ID NO:12-Sequence B-SEQ ID NO:24, SEQ ID NO:14-Sequence A-SEQ ID NO:26, and SEQ ID NO:16-Sequence C-SEQ ID NO:28.

Methods of identifying CDRs within HCVR and LCVR amino acid sequences are well known in the art and can be used to identify CDR. Typical examples used to identify CDR boundaries include, for example, the IMGT definition, the Kabat definition, the Chothia definition, the AbM definition, the Martin definition, the Gelfand definition, and the Honneger definition (Aho's definition). Public databases are also available for identifying CDR sequences within antibodies.

The present invention includes the anti-SARS-CoV-2(S) antibodies with modified glycosylation patterns. In certain embodiments, modifications can be made to remove unwanted glycosylation sites, e.g., antibodies lacking fucose moieties have enhanced antibody-dependent cellular cytotoxicity (ADCC). Also, in certain embodiments, galactosylation modifications can improve complement dependent cytotoxicity (CDC). Antibodies and the like having modifications on their sugar chains are also included in the present invention.

In addition, in one aspect, the present invention provides antibodies that compete with antibodies, containing HCVR CDRs and LCVR CDRs having amino acid sequences selected from the sequences listed in Table 1 for specific binding to the spike protein of SARS-CoV-2.

In addition, in one aspect, the present invention provides antibodies that block or reduce (preferably completely block) SARS-CoV-2 spike protein binding to ACE2. The antibodies that block or reduce (preferably completely block) binding of the SARS-CoV-2 spike protein to ACE2 may bind at or near the binding site of the SARS-CoV-2 spike protein to ACE2. In certain embodiments, the present invention provides antibodies that block or reduce (preferably completely block) binding of the SARS-CoV-2 spike protein to mammalian, preferably human ACE2.

In another aspect of the present invention, the present invention provides nucleic acid molecules encoding the anti-SARS-CoV-2 antibodies. For example, the present invention provides nucleic acid molecules encoding any of HCVR amino acid sequences listed in Table 1, and in certain embodiments, the nucleic acid molecules contain any of nucleic acid sequences encoding HCVR amino acid sequences described in Table 1, or a polynucleotide sequence selected from sequences having at least 90%, 92% or 95% sequence identity, and at least 98% or 99% sequence identity to it. The invention also provides, for example, nucleic acid molecules encoding any of LCVR amino acid sequences listed in Table 1, and in certain embodiments, the nucleic acid molecules contain any of nucleic acid sequences encoding LCVR amino acid sequences described in Table 1, or a polynucleotide sequence selected from sequences having at least 90%, 92% or 95% sequence identity, and at least 98% or 99% sequence identity to it.

In addition, the present invention provides nucleic acid molecules encoding any of HCDR1 amino acid sequences listed in Table 1, and the nucleic acid molecules contain any of nucleic acid sequences encoding HCDR1 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of HCDR2 amino acid sequences listed in Table 1, and the nucleic acid molecules contain any of nucleic acid sequences encoding HCDR2 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of HCDR3 amino acid sequences listed in Table 1, and the nucleic acid molecules contain any of nucleic acid sequences encoding HCDR3 amino acid sequences described in Table 1, or a polynucleotide sequence selected from sequences having at least 90%, 92% or 95% sequence identity, and at least 98% or 99% sequence identity to it.

In addition, the present invention provides nucleic acid molecules encoding any of LCDR1 amino acid sequences listed in Table 1, and the nucleic acid molecules contain any of nucleic acid sequences encoding LCDR1 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of LCDR2 amino acid sequences listed in Table 1, and the nucleic acid molecules contain any of nucleic acid sequences encoding LCDR2 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of LCDR3 amino acid sequences listed in Table 1, and the nucleic acid molecules contain any of nucleic acid sequences encoding LCDR3 amino acid sequences described in Table 1, or a polynucleotide sequence selected from sequences having at least 90%, 92% or 95% sequence identity, and at least 98% or 99% sequence identity to it.

In addition, the present invention provides a nucleic acid molecule encoding an HCVR amino acid sequence, wherein the HCVR amino acid sequence contains a set of three CDR amino acid sequences (i.e., HCDR1-HCDR2-HCDR3), wherein the HCDR1-HCDR2-HCDR3 amino acid sequence set is defined by any of four representative anti-SARS-CoV-2 antibodies listed in Table 1. The invention also provides a nucleic acid molecule encoding an LCVR amino acid sequence, wherein the LCVR amino acid sequence contains a set of three CDR amino acid sequences (i.e., LCDR1-LCDR2-LCDR3), wherein the LCDR1-LCDR2-LCDR3 amino acid sequence set is defined by any of representative anti-SARS-CoV-2 antibodies listed in Table 1.

In addition, the present invention provides nucleic acid molecules encoding both HCVR and LCVR, wherein the nucleic acid molecules contain nucleic acid sequences encoding any of HCVR amino acid sequences listed in Table 1 and nucleic acid sequences encoding any of LCVR amino acid sequences listed in Table 1. The nucleic acid molecules contain any of nucleic acid sequences encoding HCVR amino acid sequences listed in Table 1, or a polynucleotide sequence selected from sequences having at least 90%, 92% or 95% sequence identity and at least 98% or 99% sequence identity to it, and any of nucleic acid sequences encoding LCVR amino acid sequences listed in Table 1, or a polynucleotide sequence selected from sequences having at least 90%, 92% or 95% sequence identity and at least 98% or 99% sequence identity to it.

The antibodies of the present invention contain the anti-SARS-CoV-2(S) antibodies that bind to the same epitope as any of antibodies having amino acid sequences of HCVR and LCVR listed in Table 1 that specifically bind to the SARS-CoV-2 spike protein, or bind to at least a portion of that epitope. Such antibodies include antibodies and antigen-binding fragments thereof against the SARS-CoV-2 spike protein that cross-compete with any of antibodies having HCVR and LCVR amino acid sequences listed in Table 1.

By using methods known in the art, it can be easily determined whether antibodies (including complete antibodies and fragments thereof) bind to the same epitope as the reference anti-SARS-CoV-2(S) antibodies, or compete with the reference anti-SARS-CoV-2(S) antibodies for binding. For example, to determine if the test antibody binds to the same epitope as the reference anti-SARS-CoV-2(S) antibodies of the present invention, the reference antibodies are placed in conditions that allow them to bind to the SARS-CoV-2 spike protein under saturated conditions. The ability of the test antibody to bind to the SARS-CoV-2 spike protein is then assessed. If the test antibody is able to bind to the SARS-CoV-2 spike protein after saturation binding with the reference anti-SARS-CoV-2(S) antibodies, it is concluded that the test antibody binds to a different epitope than the reference anti-SARS-CoV-2(S) antibodies. On the other hand, if the test antibody is unable to bind to the SARS-CoV-2 spike protein after saturation binding with the reference anti-SARS-CoV-2(S) antibodies, it is concluded that the test antibody binds to the same epitope as that bound by the reference anti-SARS-CoV-2(S) antibodies. To determine if the test antibody competes with the reference anti-SARS-CoV-2(S) antibody for binding, it can be confirmed by testing for competition with each other. In a first test, the reference antibodies are allowed to bind to the SARS-CoV-2 spike protein under saturated conditions, and then the binding of the test antibody to the SARS-CoV-2 spike protein is assessed. In a second test, the test antibody is allowed to bind to the SARS-CoV-2 spike protein under saturated conditions, and then the binding of the reference antibodies to the SARS-CoV-2 spike protein is assessed. In both tests, if only the (saturated) antibody in the first test is capable of binding to the SARS-CoV-2 spike protein, then it is concluded that the test antibody and the reference antibodies compete for binding to the SARS-CoV-2 spike protein. As will be appreciated by those of skill in the art, an antibody that competes with the reference antibodies for binding will not necessarily bind to an epitope that matches the reference antibody, but will sterically inhibit the binding of the reference antibodies by binding to overlapping or adjacent epitopes. Thus, it is concluded that two antibodies bind to the same or overlapping epitopes if each competitively inhibits (blocks) the binding of the other to the antigen. Antibodies that compete with the antibodies of the present invention are also included in the present invention.

In one aspect, the present invention provides recombinant expression vectors capable of expressing the anti-SARS-CoV-2(S) antibodies. In another aspect, the present invention provides recombinant expression vectors capable of expressing polypeptides containing heavy and/or light chain variable regions of the anti-SARS-CoV-2(S) antibody. For example, the present invention contains recombinant expression vectors containing any of nucleic acid molecules described above, i.e., nucleic acid molecules encoding any of the HCVR, LCVR or CDR amino acid sequences listed in Table 1. Any expression vectors used in this technical field can be used without limitation, and such vectors are known to those skilled in the art. The antibodies of the present invention can be produced by culturing host cells into which such vectors have been introduced under conditions that allow the production of antibodies or antibody fragments, and recovering the produced antibodies and antibody fragments. Such methods are also included within the scope of the present invention.

In yet another aspect, the present invention provides a pharmaceutical composition containing a therapeutically effective amount of at least one antibody or antigen-binding fragment thereof that specifically binds to the SARS-CoV-2 spike protein, and a pharmaceutically acceptable carrier. In one embodiment, the present invention also includes a combined use of the anti-SARS-CoV-2(S) antibodies with other therapeutic agents, and a composition which is a compounding agent of the anti-SARS-CoV-2(S) antibodies and other therapeutic agents. A second therapeutic agent that is used in combination or blended with the antibodies of the present invention is any agent that is advantageously used in combination or blended with the anti-SARS-CoV-2(S) antibody. Typical agents that are advantageously used in combination or blended include, but not limited to, other agents that inhibit the activity of SARS-CoV-2 that bind to SARS-CoV-2 but do not cross-compete with the antibodies of the present invention (including other antibodies or antigen-binding fragments thereof, etc.), and/or agents that do not directly bind to SARS-CoV-2 but nevertheless inhibit viral activity, including infectivity of host cells. In certain embodiments, the second therapeutic agent is an agent that helps combat or reduce potential side effects, if any, associated with the antibodies of the present invention. The second therapeutic agents can be selected from the group consisting of, for example, anti-inflammatory agents (e.g., corticosteroids and non-steroidal anti-inflammatory agents), anti-infective agents, anti-viral agents, nutritional supplements such as antioxidants, and any other drugs and therapies known in the art. Examples thereof include preferably remdesivir, favipiravir, dexamethasone, ciclenisonide, nafamostat, camostat, ivermectin, bamilanibimab, etecevimab, casilibimab, imdevimab, and HIV protease inhibitors (e.g., lopinavir, ritonavir or combination drugs thereof, nelfinavir).

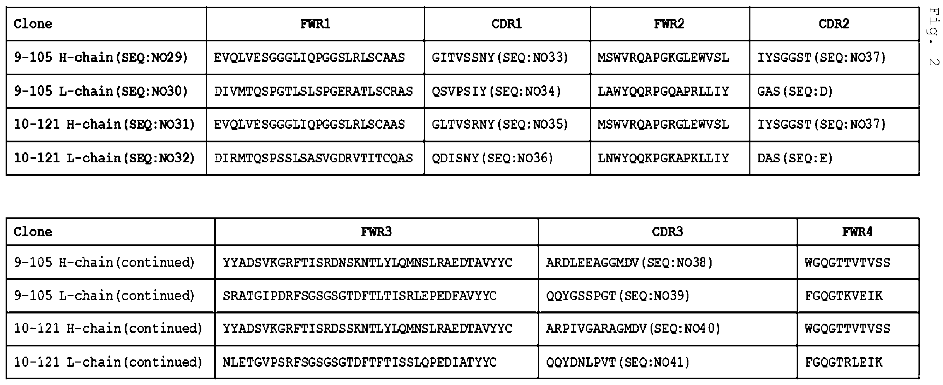

In yet another aspect, the antibodies of the present invention or its antigen-binding fragment thereof can be used in combination with other antibodies that specifically bind to the SARS-CoV-2 spike protein (so-called cocktail antibodies). When used in combination with other antibodies, it is preferable to use with an antibody having a different neutralization spectrum against SARS-CoV-2 variants than that of the antibodies of the present invention or its antigen-binding fragment thereof. For example, but not limited to, the antibodies of the present invention can be used with the 9-105 antibody or the 10-121 antibody, which have been previously reported by the inventors of the present invention (Patent Document 1, Non-Patent Document 6), or existing antibodies, such as REGN-10933 and REGN-10987 (Regeneron/Roche) (Hansen et al., Science 2020 Aug 21; 369(6506):1010-1014.), LY-CoV555 (Eli Lilly) (Jones et al., Sci Transl Med. 2021 May 12; 13(593):eabf1906.), VIR-7831 and VIR-7832 (GSK) (Pinto et al., Nature 2020 Jul; 583(7815):290-295.). The combination of antibodies is not limited to two but may consist of three or more antibodies. The antibody combinations should take into account the neutralization spectrum against SARS-CoV-2 variants. The antibodies of the present invention or its antigen-binding fragment thereof can be combined with another antibody or its antigen-binding fragment thereof, and in some cases, two or more antibodies of the present invention or their antigen-binding fragments thereof may also be used together. Preferred antibody combinations consider the neutralization spectrum against SARS-CoV-2 variants and may include the use of the antibodies of the present invention in combination with the 9-105 antibody and 10-121 antibody previously reported by the inventors. Examples include, but are not limited to, two-antibody combinations are 4-66 antibody (of the present invention) with 9-105 antibody or 1-58 antibody (of the present invention) with 10-121 antibody, three-antibody combinations are 4-66 antibody (of the present invention) with 1-58 antibody and 9-105 antibody. As a two-antibody combination, the 4-66 antibody and 1-58 antibodies of the present invention can also be used. The amino acid sequences of the heavy chain variable regions consisting of the framework regions and the CDR1-3 regions and the light chain variable regions consisting of the framework regions and the CDR1-3 regions of the anti-SARS-CoV-2 antibodies 9-105 and 10-121, which were reported by the inventors, are shown in Figure 2.

In another aspect, the present invention provides a method of treating or preventing a disease associated with SARS-CoV-2 in a subject (preferably human) using the anti-SARS-CoV-2(S) antibody or antigen-binding portion of the antibodies of the present invention. Here, the treating or preventing method includes administering a pharmaceutical composition containing a therapeutically or prophylactically effective amount of the antibodies of the present invention to a subject (preferably a human) in need thereof. In one embodiment, the present invention provides methods of preventing, treating or ameliorating at least one symptom of SARS-CoV-2 infection, including administering a therapeutically or prophylactically effective amount of the anti-SARS-CoV-2(S) antibodies of the present invention to a subject (preferably a human) in need thereof. In certain embodiments, the present invention provides a method of ameliorating or reducing the severity of at least one symptom or sign of SARS infection in a subject by administering the anti-SARS-CoV-2(S) antibodies of the present invention. At least one symptom or sign is selected from the group consisting of inflammation in lungs, alveolar damage, fever, respiratory disorders (nasal congestion, runny nose, sore throat, cough, shortness of breath), headache, malaise, dysgeusia, olfactory disturbance, diarrhea, vomiting, hypercoagulability, thrombosis, Kawasaki disease-like vasculitis, renal dysfunction (e.g., nephritis), organ failure, pneumonia, septic shock and death. In certain other embodiments, the antibodies of the present invention are administered therapeutically or prophylactically to a subject infected with or at risk of being infected with SARS-CoV-2. Subjects (patients) at risk include, but not limited to, those with immunocompromised conditions, the elderly (aged 65 and over), those with underlying medical conditions such as pulmonary infections, heart disease, respiratory disease, diabetes or dialysis patients, medical workers, persons in close contact with persons with confirmed or suspected SARS infection, or persons expected to have contact with such persons. The antibodies of the present invention are administered intradermally, transdermally, intramuscularly, intraperitoneally, intravenously, subcutaneously, intranasally, epidurally, or orally. In one preferred embodiment, the antibodies of the present invention are administered as a single intravenous injection, taking into account the maximum concentration of the antibody in the subject's (patient's) plasma. In one embodiment, the antibodies of the present invention are administered at a dose of about 0.1 mg to about 100 mg/kg body weight of the subject. In certain other embodiments, the antibodies of the present invention are administered once or multiple times at a dose containing about 0.1 mg to about 100 mg/kg body weight of the subject.

The present invention also includes the use of the anti-SARS-CoV-2(S) antibody or antigen-binding fragment thereof of the present invention in the production of a medicament.

An antibody can be used in the sense of including immunoglobulin molecules containing four polypeptide chains, and two heavy (H) chains and two light (L) chains inter-connected by disulfide bindings (i.e., "complete antibody molecules"), multimers thereof (e.g., IgA or IgM), or antigen-binding fragments thereof. Each heavy chain of the antibody contains a heavy chain variable region (HCVR or VH) and a heavy chain constant region (including domains CH1, CH2 and CH3). Each light chain of the antibody contains a light chain variable region (LCVR or VL) and a light chain constant region (CL). HCVR and LCVR are further subdivided into framework regions (FR) and complementarity determining regions (CDR). Each HCVR and LCVR contains three CDRs and four FRs, arranged from amino-terminus to carboxy-terminus in the following order: FR1, CDR1, FR2, CDR2, FR3, CDR3, FR4. In embodiments of the antibodies of the present invention, the FRs of the antibody (or antigen-binding fragment thereof) may be identical to human germline sequences, or may be naturally or artificially modified.

An antibody can also have one or more CDR residues substituted or one or more CDR residues omitted or added without losing its binding properties. Antibodies have also been reported that bind even with the omission of one or two CDR residues. CDR residues that do not contact with an antigen can be identified by molecular modeling or experimentally. When substitutions of CDR residues are made, they may preferably be made at residues that do not contact with an antigen. The amino acid residues to be substituted and the positions for substitution within the CDRs are selected empirically based on a variety of factors. Substitutions can be conservative or non-conservative amino acid substitutions. A "conservative amino acid substitution" is one in which an amino acid residue is replaced by another amino acid residue having a side chain (R group) with similar chemical properties (e.g., charge or hydrophobicity), and in general, conservative amino acid substitutions do not substantially alter the functional properties of a protein (e.g., antigen-binding activity or binding properties). Examples of groups of amino acids with side chains with similar chemical properties include: 1) aliphatic side chains: glycine, alanine, valine, leucine and isoleucine; 2) aliphatic-hydroxyl side chains: serine and threonine; 3) amide-containing side chains: asparagine and glutamine; 4) aromatic side chains: phenylalanine, tyrosine and tryptophan; 5) basic side chains: lysine, arginine and histidine; 6) acidic side chains: aspartate and glutamate; and 7) sulfur-containing side chains: cysteine and methionine. Conservative amino acid substitutions refer to substitutions between amino acids within the same class, and non-conservative amino acid substitutions refer to exchanging members of one of these classes for members of another class. Preferred conservative amino acid substitutions are between: valine-leucine-isoleucine, phenylalanine-tyrosine, lysine-arginine, alanine-valine, glutamate-aspartate, and asparagine-glutamine.

The human anti-SARS-CoV-2(S) antibodies disclosed in the present specification include those having one or more amino acids substituted, added and/or deleted in the framework and/or CDR regions of the heavy and light chain variable domains compared to the corresponding sequences. The present invention includes antibodies and antigen-binding fragments thereof derived from any of amino acid sequences disclosed in the present specification, wherein within one or more framework and/or CDR regions, one or more amino acids may be conservatively substituted or mutated to the corresponding residues in the germline sequence from which the antibody is derived, or to the corresponding residues in another human germline sequence (such sequence alterations are referred to herein as "conservative mutation"). One skilled in the art can generate numerous antibodies and antigen-binding fragments containing one or more individual mutations or combinations thereof starting from the heavy and light chain variable region sequences disclosed in the present specification. The resulting antibodies and antigen-binding fragments can be readily identified and selected for one or more desired properties, such as improved binding specificity, increased binding affinity, reduced immunogenicity, and the like. Antibodies and antigen-binding fragments obtained by such general methods are also included in the present invention.

In one aspect, the amino acid substitutions for the anti-SARS-CoV-2(S) antibodies of the present invention give any one or more of (1) reducing susceptibility to proteolysis, (2) reducing susceptibility to oxidation, (3) changing binding affinity to form protein complexes, and (4) conferring or altering other physicochemical or functional properties, and are amino acid substitutions conserving specific binding to the SARS-CoV-2 spike protein. For example, one or more amino acid substitutions, preferably conservative amino acid substitutions, may be made in parts of the polypeptide, preferably outside the domains that form intermolecular contacts between the antigen and the antibody. Conservative amino acid substitutions should not substantially alter the structural features of the parent sequence; and for example, the substituted amino acids should not alter the antiparallel β-sheets that make up the immunoglobulin binding domain occurring in the parent sequence, or should not disrupt other secondary structures that characterize the parent sequence. Examples of secondary and tertiary structures of polypeptides known to those skilled in the art are described in, for example, Proteins, Structures and Molecular Principles (Creighton, Ed., W.H. Freeman and Company, New York (1984)); Introduction to Protein Structure (C. Branden and J. Tooze, eds., Garland Publishing, New York, N. Y. (1991)); and Thornton et al., Nature 354: 105 (1991), which are incorporated herein as a part of the present specification.

In addition, the present invention also includes the human anti-SARS-CoV-2(S) antibodies including variants of any of HCVR, LCVR or CDR amino acid sequences disclosed in the present specification, having one or more conservative substitutions. For example, the present invention includes the anti-SARS-CoV-2(S) antibodies having no more than 5, no more than 8, no more than 4, no more than 3, no more than 2, no more than 1 conservative amino acid substitutions related to any of HCVR, LCVR or CDR amino acid sequences disclosed in the present specification.

The antibody in the present invention is preferably a human antibody. The human antibody refers to an antibody that has variable and constant regions derived from human germline immunoglobulin sequences. The antibodies exemplified in the present specification are human antibodies generated by in vitro activating B cells taken from patient donors who have recovered from SARS-CoV-2 infection. As used herein, "human antibody" is not intended to include monoclonal antibodies in which CDR sequences derived from the germline of another mammalian species (e.g., mouse) have been grafted onto human FR sequences, although it is meant to include antibodies produced recombinantly in non-human mammals or non-human mammalian cells. The antibodies described in examples of the present specification are human antibodies.

As used herein, "recombinant" means the antibodies of the present invention produced, expressed, isolated, or obtained by techniques or methods known in the art as recombinant DNA technology, including, for example, transgenic expression; and includes an antibody or antigen-binding fragment thereof expressed in a non-human mammal (including a transgenic non-human mammal, e.g., transgenic mouse), or mammalian cell (e.g., CHO cell) expression system, or isolated from recombinant combinatorial human antibody libraries.

As used herein, an expression such as "specifically binding" means that an antibody or an antigen-binding fragment thereof binds to an antigen under physiological conditions to form a complex. Specific binding is characterized by an equilibrium dissociation constant of at least about 1×10⁻⁸ M or less. Methods for determining whether two molecules specifically bind are well known in the art and include, for example, equilibrium dialysis, surface plasmon resonance, and the like, and it can be confirmed using these methods as appropriate. The surface plasmon resonance is identified, for example, by BIACORE (trademark).

As used herein, the terms "antigen-binding fragment" of an antibody, "antigen-binding fragment thereof" of an antibody, "fragment" of an antibodies, include polypeptides or glycoproteins that specifically bind to the antigen SARS-CoV-2 spike protein to form a complex, that are optionally naturally occurring, obtained using enzymatic or biochemical means, synthesized, or obtained by genetic engineering. As used herein, the term refers to one or more fragments of an antibody that retains the ability to bind to the SARS-CoV-2 spike protein, and for example, may be produced by recombinant DNA techniques or enzymatically or chemically cleaving a complete antibody. As used herein, the term includes, but not limited to, for example, Fab, Fab', F(ab')2, Fd, Fv, disulfide Fv, dAb fragments, fragments containing complementarity determining regions (CDRs), single domains such as isolated CDR fragments, restricted FR3-CDR3-FR4 peptides, single chain antibodies (scFv), minibodies, nanobodies (e.g., monovalent nanobodies, bivalent nanobodies, etc.), diabodies (diabody), domain deficient antibodies, chimeric antibodies, CDR-grafted antibodies, bispecific antibodies, multispecific antibodies (e.g., trispecific antibody, tetraspecific antibody), polypeptides containing at least a portion of an antibody having specific antigen-binding ability, and other engineered molecules such as small modular immunodrugs.

Antigen-binding fragments of antibodies can be obtained using any suitable standard technique, such as, for example, protein digestion, or recombinant genetic engineering techniques involving the manipulation and expression of the DNA encoding the variable and (optionally) constant domains of the antibody, from a complete antibody molecule or its amino acid or genetic information. DNA is sequenced and chemical or molecular biological techniques are used, for example, to place one or more variable and/or constant domains in proper arrangement, alternatively, for example, arbitrary codons are introduced to create cysteine residues or to modify/add/delete amino acids, and other operation are performed, thereby any antigen-binding fragment can be produced. It is not limited to, but Fab, Fv, and scFv antibody fragments can all be expressed in E. coli and secreted, making them easy to produce in large quantities. Alternatively, Fab'-SH fragments can be expressed in E. coli, recovered, and chemically coupled to generate F(ab')₂ fragments. Additionally, F(ab')₂ fragments can be expressed in recombinant host cells and isolated from the culture medium. Furthermore, using previously reported techniques, Fv fragments and single-chain Fv (scFv) fragments can also be produced. Fv and scFv are antibody fragments that lack the constant region but retain a complete antigen-binding site. Additionally, an effector protein can be fused to either the N-terminus or C-terminus of scFv, creating an scFv fusion protein. Various technologies for producing antibody fragments are well known, and any of these techniques can be used in the present invention without limitation.

An antigen-binding fragment of an antibody typically contains at least one variable domain. A variable domain can be of any size or amino acid composition and generally contains at least one CDR, which is flanked by or in-frame with one or more framework sequences. In antigen-binding fragments having a VH domain joined to a VL domain, the VH and VL domains are relatively positioned in any suitable arrangement. Antigen-binding fragments of antibodies include, for example, variable regions that are dimeric, VH-VH, VH-VL or VL-VL dimers. Alternatively, antigen-binding fragments of antibodies contain monomeric VH or VL domains.

In certain embodiments, an antigen-binding fragment of an antibody contains at least one variable domain covalently linked to at least one constant domain. Typical arrangements of variable and constant domains within the antigen-binding fragments of antibodies of the present invention include, but are not limited to: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any arrangement of variable and constant domains, including any of exemplary arrangements described above, the variable and constant domains are either directly linked to each other or linked by a linker region. The linker region consists of at least 2 (e.g., 5, 10, 15, 20, 40, 60 or more) amino acids and connect adjacent variable and/or constant domains. Additionally, the antigen-binding fragments of antibodies of the present invention contain homo-dimers or hetero-dimers (or other multimers) of any of the above variable and constant domain arrangements in which one or more monomeric VH or VL domains are non-covalently associated.

As with complete antibody molecules, antigen-binding fragments can be monospecific or multispecific (e.g., bispecific). A multispecific antigen-binding fragment of an antibody typically contains at least two different variable domains, each variable domain has an ability of specifically binding to different antigens or different epitopes on the same antigen. Any multispecific antibodies, including the bispecific antibodies in the present specification, can be produced using routine techniques available in the art and used as antigen-binding fragments of antibodies for the purposes of the present invention.

The antibodies of the present invention are preferably an isolated antibody. As used herein, the "isolated antibody" means an antibody that is substantially free from other antibodies with different antigenic specificities.

As used herein, the terms "neutralizing antibody", "antibody that neutralizes", "antibody exhibiting neutralizing activity", and "antibody having an ability of neutralizing", etc. refer to an antibody wherein binding of the antibody to the SARS-CoV-2 spike protein results in inhibition of at least one biological activity of SARS-CoV-2, wherein the antibodies of the present invention prevent (reduce) or block binding of SARS-CoV-2 to ACE2, or prevent (reduce) or block infection of cells via binding of SARS-CoV-2 to ACE2 (i.e., inhibit cell membrane fusion). Preferably, the antibodies of the present invention completely block the binding of SARS-CoV-2 to ACE2.

The antibodies of the present invention have an ability of immunospecifically binding to the SARS-CoV-2 spike protein and neutralizing SARS-CoV-2 viral infection. The activity of the antibodies of the present invention is determined by in vitro or in vivo assays. The activity can be determined and measured based on the binding activity to the SARS-CoV-2 spike protein, preferably RBD of the spike protein. The activity can also be confirmed and measured by neutralizing activity against the SARS-CoV-2 virus. Preferably, the activity is measured by neutralizing activity. The ability of the antibodies of the present invention to bind SARS-CoV-2 and neutralize the activity of SARS-CoV-2 is measured using any standard methods known to those of skill in the art, including binding assays or neutralization assays, as described herein. Representative in vitro assays for measuring binding and blocking activity are described in examples of the present specification. The term "50% inhibitory concentration" (abbreviated as "IC₅₀") represents the concentration of the antibodies of the present invention required for 50% neutralization of the SARS-CoV-2 virus. Lower IC₅₀ values have stronger neutralizing activity.

The term "TCID₅₀" refers to the amount of virus required to infect 50% of the cells in the tissue culture. 100x and 200x refer to 100 or 200 times the concentration of virus compared to TCID₅₀.

In certain embodiments, the antibodies of the present invention have a neutralizing ability expressed as 50% inhibitory concentration (IC₅₀ µg/ml) at the antibody concentrations within a range of about 0.01 µg/mL to about 1.0 µg/mL, or about 0.01 µg/mL to about 0.5 µg/mL, or about 0.02 µg/mL to about 0.25 µg/mL in neutralizing the SARS-CoV-2 virus in the neutralization assay using a pseudovirus. The type of target cells used in the neutralization assay using a pseudovirus is not particularly limited, and any cells can be used as long as virus infection can be detected. Examples thereof include, but are not limited to, artificially produced cells (293F/ACE2/TMPRSS2 cells), human lung cancer cells (alveolar epithelial cells, Calu-3), and gastrointestinal tract mucosal epithelial cells (Caco-2). Since the neutralizing activity may differ depending on the target cell, for example, the 50% inhibitory concentration can be defined as the neutralizing ability when using 293F/ACE2/TMPRSS2 cells. Antibodies used in the neutralization assays described in examples in the present specification show the 50% inhibitory concentration at about 0.02 µg/ mL against SARS-CoV-2 prototype (614G). This indicates that the antibodies of the present invention have strong neutralizing activity.

In certain embodiments, the antibodies of the present invention have a neutralizing ability against SARS-CoV-2 virus variants. Mutant strains of the SARS-CoV-2 virus have mutations in any part of the sequence of SARS-CoV-2 virus, and especially those with mutations in spike protein sequence can be exemplified. The variants include, but are not limited to, for example, Alpha strain (B.1.1.7), Beta (B.1.351), Gamma (P.1), Delta (B.1.617.2), Kappa (B.1.617.1), Lambda (C.37), and Mu (B.1.621), etc. The antibodies of the present invention preferably exhibit neutralizing ability against many SARS-CoV-2 variant strains in a neutralization assay using a pseudovirus, with a 50% inhibitory concentration (IC₅₀) in the range of about 0.02 µg/mL to about 1 µg/mL, about 0.02 µg/mL to about 0.8 µg/mL, about 0.02 µg/mL to about 0.5 µg/mL, about 0.02 µg/mL to about 0.2 µg/mL, or about 0.02 µg/mL to about 0.1 µg/mL. Furthermore, in the neutralization assay described in the examples of this specification, the antibodies used demonstrated a 50% inhibitory concentration (IC₅₀) in the range of about 0.05 µg/mL to about 0.25 µg/mL against the Omicron variant and its subvariants.

As used herein, the term "epitope" refers to a site or region on an antigen that is an antigenic determinant that interacts with a specific antigen-binding site in the variable region of an antibody molecule. The epitopes is defined as either structural or functional. A functional epitope is generally a subset of a structural epitope and includes those residues that are directly involved in the affinity of the interaction. The epitope can also be conformational, including non-linear amino acid relationships. In certain embodiments, the epitope includes determinants that are chemically active surface groups of molecules such as amino acids, sugar side chains, phosphate groups or sulfonyl groups, and for example, has specific three dimensional structural features and/or specific residue modifications. When amino acid substitutions are made in the antibodies of the present invention, substitutions that do not affect the epitope-binding activity of the antibody or antigen-binding fragment thereof or that have little effect on the binding activity are preferred.

As used herein, the term "cross-compete" means that an antibody or antigen-binding fragment thereof binds to an antigen and inhibits or blocks the binding of another antibody or antigen-binding fragment thereof. In certain embodiments, the first and second antibodies bind to the same epitope, thereby inhibiting or blocking the binding of the other antibody. Alternatively, the first and second antibodies bind to different but overlapping epitopes such that the binding of one antibody inhibits or blocks binding of the other antibody, e.g., through steric hindrance. Cross-competition between antibodies can be measured by methods known in the art, such as surface plasmon resonance and biolayer interferometry assay.

When we refer herein to "substantial identity" or "identity" of amino acid sequences of an antibody or antigen-binding fragment thereof, it means identity when the two amino acid sequences are optimally aligned, for example, by the programs GAP or BESTFIT using default gap weights. Non-identical amino acid residues in the compared sequences preferably differ between the sequences by conservative amino acid substitution. Amino acid sequence identity can be determined using sequence analysis software. The protein analysis software matches similar sequences using measures of similarity assigned to various substitutions, deletions, and other modifications, including conservative amino acid substitution. For example, analysis is performed using GCG software (e.g., GCG Version 6.1), FASTA (e.g., FASTA2 or FASTA3), BLASTP or TBLASTN.

When referring to "sequence identity" or "identity" in a nucleic acid sequence, it is optimally aligned and compared to another nucleic acid (or its complementary strand) with appropriate nucleotide insertion or deletion, and for example, it is measured by any well-known algorithm for sequence identity, such as FASTA, BLAST, or GAP.

As used herein, the term "subject" refers to animals infected or at risk of being infected with a virus and in need of amelioration, prevention, and/or treatment for a disease or condition such as a viral infection, preferably, mammals, more preferably humans. In certain embodiments, the subject is a human infected with or at risk of being infected with SARS-CoV-2.

The antibodies of the present invention specifically bind to the SARS-CoV-2 spike protein. Therefore, the antibodies of the present invention can also be used to detect the antigen SARS-CoV-2 virus or the SARS-CoV-2 spike protein.

"Binding affinity", which describes the binding activity of an antibody and an antigen, generally means power which is the sum of non-covalent interactions between a single binding site of a molecule (e.g., antibody) and its binding partner (e.g., antigen). "Binding affinity" is expressed as a unique numerical value that reflects a 1:1 interaction between members of a binding pair (e.g., antibody and antigen). The affinity of molecule X for its partner Y can generally be expressed by the equilibrium dissociation constant (Kd), calculated as the ratio koff/kon. See, e.g., Chen, et al. (1999) J. Mol Biol 293: 865-881. Affinity can be measured by common methods known in the art. Low-affinity antibodies generally bind an antigen slowly and tend to dissociate easily, whereas high-affinity antibodies generally bind an antigen more quickly and tend to keep the bound state longer. Examples of measurement methods include, but are not limited to, radiolabeled antigen binding assays (RIA), ELISA-based immunoassays, and surface plasmon resonance assay using Biacore (registered trademark). Methods and reagents suitable for determining the binding properties of the antibody or antigen-binding fragment thereof or altered/mutated derivative thereof of the present invention are known in the art and commercially available. In addition, instruments and software designed for such analyzes are also commercially available (e.g., Biacore (registered trademark) A100, and Biacore (registered trademark) 2000).

In certain embodiments, the antibody-antigen binding assay may be performed either as a direct binding assay or as competitive binding assay. Binding can be detected using standard ELISA or standard flow cytometric assays. In direct binding assays, a test antibody is tested for binding to its antigen, while in competitive binding assays, the ability of a test antibody to compete with known antibodies that bind to the SARS-CoV-2 spike protein is assessed. These methods are used to screen for those that provide the desired properties.

Antibodies specific to the SARS-CoV-2 spike protein can also be further labeled. Labels can be attached to the N-terminus or C-terminus of the antibody, or to the side chains of any amino acid residue. Labels are, for example, avidin-biotin, radionuclides, fluorescent dyes, or MRI-detectable labels. In certain embodiments, labeled antibodies are used in diagnostic assays, including imaging assays.

In addition to the amino acid sequences of the antibodies of the present invention described in the present specification, the present invention also provides nucleotide sequences that correspond to and encode the amino acid sequences. Accordingly, the present invention also includes an isolated nucleic acid having a nucleotide sequence encoding the antibodies of the present invention. The isolated nucleic acid is preferably cDNA. These nucleic acid sequences include nucleic acid sequences encoding part or all of the light and heavy chains and CDRs of the antibody of the present invention and these are also included in the present invention. Accordingly, the present invention also contains polynucleotide sequences encoding the VH and VL framework regions, including the CDRs and FRs, of the antibodies described in the present specification, and expression vectors for their efficient expression in cells (e.g., mammalian cells). Methods for producing antibodies using polynucleotides are known in the art, and those methods can be used as appropriate. Methods of making the antibody of the present invention using these methods are also included in the present invention.

Exemplary techniques of the production of antibodies that can be used in the present invention are described below. The antibodies of the present invention can be prepared using a wide range of techniques known in the art, including genetic recombination techniques, phage display techniques, or combinations thereof.

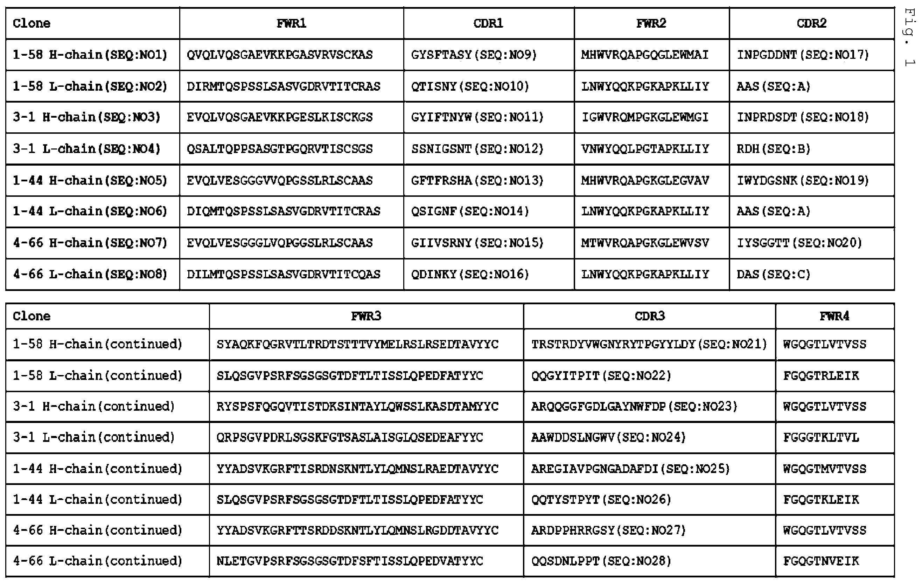

Production and screening methods for specific antibodies using gene recombination technologies are well known and commonly used techniques in the art. A DNA encoding the amino acid sequence of the antibodies of the present invention can be easily prepared using known techniques. For example, by referring to the sequences of SEQ ID NOs listed in Figure 1, DNA sequences encoding the amino acids of each CDR can be chemically synthesized and combined arbitrarily, though the means is not limited to this. At that time, it can be combined with any DNA encoding the amino acid sequence of VH or VL, FR.

For expression of an antibody or antigen-binding fragment thereof, or a variant thereof, an expression vector containing polynucleotides encoding the antibodies is constructed. Thus, replicable vectors containing nucleotide sequences encoding an antibody molecule, a heavy chain or light chain of an antibody, a variable domain of a heavy or light chain of an antibody or portion thereof, or any CDR of a heavy or light chain, operably linked to a promoter, are provided, and also included in the present invention. Such vectors can contain nucleotide sequences encoding the constant regions of the antibody molecule, and variable domains of the antibodies may also be cloned into vectors for expression of whole heavy chain, whole light chain, or both whole heavy and light chains. The expression vector is introduced into host cells and the transfected cells are then cultured to produce the antibody. Accordingly, the present invention includes host cells containing polynucleotides encoding amino acid sequences of the antibodies of the present invention. For expression of double-chain antibodies, vectors encoding both heavy and light chains may be expressed simultaneously in a host cell for expression of the entire immunoglobulin molecule.

Mammalian cell strains available as hosts for the expression of recombinant antibodies include many immortalized cell strains known in the art and available from the ATCC, including but not limited to, for example, Chinese Hamster Ovary (CHO) cells, HeLa cells, baby hamster kidney (BHK) cells, monkey kidney cells (COS), human hepatocellular carcinoma cells (e.g., HepG2), human renal epithelial cells (HREpC), human embryonic kidney cells (HEK), and numerous other cell strains. Appropriate cell strains or host systems can be chosen to ensure the correct modification and processing of the antibody or portion thereof expressed. For this reason, eukaryotic host cells that have the cellular machinery for proper primary transcript processing, glycosylation and phosphorylation of the gene product are preferably used. Such mammalian host cells include, but are not limited to, CHO, VERY, BHK, HeLa, COS, MDCK, HEK293, 3T3, W138, BT483, Hs578T, HTB2, BT2O, T47D, NS0 (mouse myeloma cell strain that does not endogenously produce any functional immunoglobulin chains), SP20, CRL7O3O and HsS78Bst cells. Further, an antibody can be produced using human cell strains developed by immortalizing human lymphocytes using genetic recombination technologies, and in addition, a human cell strain PER. C6. can also be used to produce an antibody.

Other cell strains that can be used as hosts for the expression of recombinant antibodies also include, for example, insect cells (e.g., Sf21/Sf9, Trichoplusiani Bti-Tn5b1-4) or yeast cells (e.g., S. cerevisiae, Pichia et al.), plant cells, and chicken cells.

The transfected cell strain is maintained in cell culture media under conditions known in the art to confer antibody expression and production. Media and culture techniques known in the art can be used without limitation. Antibody production can be carried out in large quantities by bioreactor processes using fed-batch, batch, perfusion, or continuous feed bioreactor methods known in the art, and such techniques enable the production of large quantities of antibodies.

The present invention is also a method for producing the antibodies of the present invention. The production method of the present invention includes a step of culturing the host cell of the present invention under conditions suitable for expression of an antibody or antigen-binding fragment thereof. Such methods may further include a step of isolating an antibody or antigen-binding fragment thereof from the host cell culture and optionally a step of blending the isolated antibody or fragment thereof into a composition.

Antibodies or antigen-binding fragments thereof or amino acid-substituted forms thereof can also be produced using a so-called phage display technology. A phage display library can be constructed with reference to the sequences described herein, and binding to the SARS-CoV-2 spike protein can be used as an index to generate an antibody or antigen-binding fragment thereof of the present invention. In the phage display method, functional antibody domains are displayed on the surface of phage particles which carry the polynucleotide sequences encoding them. A phage expressing an antigen binding domain that binds to the antigen of interest can be selected or identified using, for example, a labeled antigen or an antigen bound or captured to a solid surface or bead.

### Antibody purification and isolation

After being produced by expression using recombinant techniques, the antibody molecule may be purified by any method known in the art for purifying immunoglobulin molecules, for example, by chromatography (e.g., ion exchange chromatography, affinity chromatography, in particular by affinity to specific antigen protein A or protein G, and size exclusion column chromatography), centrifugation, difference in solubility, or any other standard protein purification techniques. Additionally, the antibodies of the present invention may be fused to heterologous polypeptide sequences (commonly referred to as "tags") known to facilitate purification.

Using recombinant techniques, the antibody can be produced intracellularly, in the periplasmic space, or directly secreted into a medium. If the antibody is produced intracellularly, as a first step, the particulate debris, either host cells or lysed fragments, is removed, for example, by centrifugation or ultrafiltration. If the antibody is secreted into a medium, the supernatant from the expression system is concentrated using protein concentration filters, for example, an ultrafiltration unit.

Antibody-containing preparations prepared from cells may be purified, for example, by hydroxylapatite chromatography, hydrophobic interaction chromatography, ion-exchange chromatography, gel electrophoresis, dialysis, and/or affinity chromatography, used alone or in combination with other purification steps. If the antibody contains a CH3 domain, Bakerbond ABX resin is useful for purification. Other protein purification techniques, for example, fractionation on ion exchange columns, ethanol precipitation, reverse phase HPLC, silica chromatography, heparin chromatography, anion or cation exchange resin (such as polyaspartic acid columns) sepharose chromatography, chromatofocusing, SDS-PAGE, and ammonium sulfate precipitation are also available, depending on the antibody to be recovered.

In certain embodiments, substantially purified or isolated antibodies of the present invention are provided. In certain embodiments, such purified or isolated recombinantly expressed antibodies can be administered to a patient to achieve a prophylactic or therapeutic effect.

Human antibodies can be produced using methods well known in the art. Human antibodies avoid some of the problems associated with antibodies with mouse or rat variable and/or constant regions. The presence of such mouse or rat derived proteins can cause rapid clearance of the antibodies or can cause the patient to develop an immune response against the antibodies.

Human antibodies can also be obtained by in vitro methods. For example, a phage display method, a ribosome display method and a yeast display method are mentioned. Human antibodies or antibody fragments can be made in vitro from immunoglobulin variable (V) domain gene repertoires derived from unimmunized donors by using a phage display technology (see, e.g., US Pat. No. 5,969,108). Alternatively, human antibodies may also be generated by in vitro activated B cells (see, e.g., US Pat. Nos. 5,567,610 and 5,229,275).

Immunoglobulin genes undergo various modifications during the maturation of the immune response, including recombination between V, D and J gene segments in the variable regions, isotype switching, and hypermutation. Although recombination and somatic hypermutation result in antibody diversity and affinity maturation, they can also increase the immunogenicity risk of antibodies. In general, mutations in CDR regions appear to contribute to improved affinity and function, while mutations in framework regions can increase immunogenicity risk. Thus, framework sequences can reduce risk by making them germline similar. It is desirable to remove potential structural factors that could lead to instability, aggregation, heterogeneity, or increased immunogenicity of the antibody produced. Examples of undesirable structural factors include unpaired cysteines (which can lead to unwanted disulfide binding formation or variable sulfhydryl adduct formation), N-linked glycosylation sites (structure and activity resulting in heterogeneity), as well as deamidation (e.g., NG, NS), isomerization (DG), oxidation (exposed methionine), and hydrolysis (DP) sites. Therefore, to reduce the risk of immunogenicity and improve pharmaceutical properties, it is desirable to revert framework sequences to germline, revert CDRs to germline, and/or remove structural factors. Thus, in certain embodiments, where a particular antibody differs from its respective germline sequence at the amino acid level, the antibody sequences may be backmutated to the germline sequence. Such corrective mutations can occur at one, two, three or more positions, or at any combination of mutated positions, using standard molecular biology techniques. Therefore, antibodies or antigen-binding fragments thereof having such mutable sequences can also be included in the present invention. In addition, the antibodies of the present invention can be subjected to mutations and amino acid substitutions from such viewpoints, and these are also included in the present invention.

In one embodiment, the antibody of the present invention is an antibody fragment or an antibody containing a fragment thereof. The antibody fragment includes a portion of a complete antibody that is the antigen-binding or variable region thereof. Examples of the antibody fragment include, but are not limited to, Fab, Fab', F(ab')2, Fd, Fv fragments, disulfide Fv, single chain antibodies (ScFv), single domain antibodies, nanobodies, and diabodies.

In another embodiment, the antibody of the present invention is a domain antibody, e.g., an antibody containing small functional binding units of antibodies corresponding to the heavy chain variable (VH) or light chain variable (VL) regions of a human antibody. Examples of the domain antibody include, but are not limited to, antibodies produced using Domantis technology (e.g., WO 04/058821; WO 04/081026; WO 04/003019; WO 03/002609).

In certain embodiments, the antibody of the present invention is a linear antibody. The linear antibody contains a pair of tandem Fd segments (VH-CH1-VH-CH1) that form a pair of antigen-binding regions.

In certain embodiments, the antibody of the present invention can be monospecific, bispecific, or multispecific. The multispecific antibody is either specific for different epitopes of a single target polypeptide, or contains antigen-binding domains specific for multiple target polypeptides. Any of the multispecific antigen-binding molecules of the present invention, or variants thereof, can be constructed using molecular biology techniques known to those skilled in the art (e.g., recombinant DNA and protein expression techniques).

In certain embodiments, the SARS-CoV-2 spike protein-specific antibody that is a bispecific antibody can be made by conjugating variable regions that bind separate domains of the SARS-CoV-2 spike protein into a single binding molecule to be dual-domain specific. Properly designed and engineered bispecific antibodies can totally enhance the SARS-CoV-2 virus inhibitory activity through increased specificity and binding activity. In one example of bispecificity, a single VL segment (e.g., VL1) is combined with two different VH domains (e.g., VH1 and VH2) to provide bispecificity including two binding "arms" (VH1-VL1, and VH2-VL1). Using a single VL segment can reduce system complexity and increase efficiency in cloning, expression, and purification methods.

Other typical bispecificity formats include, but are not limited to, scFv-based bispecificity formats. The bispecificity formats of bispecific antibodies include, for example, IgG-scFv fusions, dual variable domain (DVD)-Ig, quadroma, knobs-into-holes, common light chains (e.g., common light chains having knobs-into-holes, etc.), CrossMab, CrossFab, (SEED) body, Leucine Zipper, Duobody, IgG1/IgG2, Dual Action Fab (DAF)-IgG, and Mab2 bispecificity formats. The bispecific antibodies can also be constructed using peptide/nucleic acid linkages.

The present invention also includes further modified forms of the antibodies of the present invention, and variants and fragments thereof, including substitutions, additions and/or deletions, and post-translational modifications of one or more amino acid residues and/or polypeptides in the variable light chain (VL) domain and/or the variable heavy chain (VH) domain and the Fc region. The modified forms also include antibody conjugates, in which other entities, such as proteins, peptides, drugs, or labels are covalently attached to the antibody. Such antibody alterations and modifications can be used to alter the biochemical and/or functional properties of the antibody such that they are suitable for treating and/or diagnosing SARS-CoV-2 infection. Techniques for making such alterations and modifications are known in the art and can be used without limitation in the present invention.

In certain embodiments, antibody alterations can be made by one or more amino acid alterations (e.g., substitutions, deletions and/or additions) introduced into one or more of the variable regions of the antibody. In another embodiment, amino acid alterations may be introduced into the framework regions. The altered antibodies can be screened using its activity as an index using the methods described in the present specification. Amino acid substitutions may be conservative or non-conservative as described above. Also, if desired, unnatural amino acids or amino acid analogs can be substituted or added into the antibody sequence. Examples of such amino acids include, but are not limited to, D-isomers of natural amino acids, α-aminoisobutyric acid, 4-aminobutyric acid, Abu, 2-aminobutyric acid, γ-Abu, ε-Ahx, 6-Aminohexanoic acid, Aib, 3-aminopropionic acid, ornithine, norleucine, norvaline, hydroxyproline, sarcosine, citrulline, cysteic acid, t-butylglycine, t-butylalanine, phenylglycine, cyclohexylalanine, β-alanine, fluoroamino acids, designer amino acids such as β-methyl amino acids, Cα-methyl amino acids, Nα-methyl amino acids, and other amino acid analogs.

In another embodiment, in the antibodies of the present invention, any cysteine residue not involved in maintaining the proper conformation of the antibody or antigen-binding fragment thereof may be replaced with, for example, serine, thereby increasing the oxidation stability of the molecule and preventing abnormal cross-linking. Also, one or more cysteine linkages can be added to the antibody to improve its stability. In particular, it is useful when the antibody or the like is an antibody fragment such as an Fv fragment.

In certain embodiments, the antibodies of the present invention can be bound (conjugated or covalently bonded) to other substances using methods known in the art. The substances to be bound include therapeutic agents, therapeutic adjuvants, detectable labels or solid supports. The substances to be bound to antibodies include, but are not limited to, amino acids, peptides, proteins, polysaccharides, nucleosides, nucleotides, oligonucleotides, nucleic acids, haptens, drugs, hormones, lipids, lipid aggregates, synthetic polymers, microparticles, living cells, viruses, fluorophores, chromophores, dyes, toxins, haptens, enzymes, antibodies, antibody fragments, radioisotopes, solid matrix, semi-solid matrix and combinations thereof. Methods for binding these other substances to antibodies are known in the art and can be used without limitation in the present invention.

In one embodiment, the antibodies of the present invention are bound to a solid support. Antibodies bound to solid supports can be used, for example, as part of screening, purification, manufacturing processes, diagnostic methods or compositions. Solid supports are generally substantially insoluble in liquid phases. Numerous supports are well known to those of skill in the art and can be used in the present invention. The solid supports include, but are not limited to, solid and semi-solid matrix such as aerogels and hydrogels, resins, beads, biochips (including thin film-coated biochips), microfluidic chips, silicon chips, multi well plates (also called microtiter plates or microplates), membranes, conductive and non-conductive metals, glass (including microscope slides) and magnetic supports. Examples of more specific solid supports include silica gel, polymeric membranes, particles, derivatized plastic films, glass beads, cotton, plastic beads, alumina gels, polysaccharides such as sepharose, polyacrylates, polystyrene, polyacrylamides, polyols, agarose, agar, cellulose, dextran, starch, FICOLL, heparin, glycogen, amylopectin, mannan, inulin, nitrocellulose, diazocellulose, polyvinyl chloride, polypropylene, polyethylene (including polyethylene glycol), nylon, latex beads, magnetic beads, paramagnetic beads, superparamagnetic beads, and the like.

In certain embodiments, the solid support may have a reactive functional group for binding to the antibodies of the present invention, such as a hydroxyl group, a carboxyl group, an amino group, a thiol group, an aldehyde group, a halogen group, a nitro group, a cyano group, an amide group, a urea group, a carbonate group, a carbamate group, an isocyanate group, a sulfone group, a sulfonate group, a sulfonamide group, a sulfoxide group, and the like.

In certain embodiments, the antibody of the present invention can be bound to labels for diagnostic and other measurement purposes (e.g., detection of SARS-CoV-2 virus). Labels used in binding to antibodies include, but are not limited to, chromophores, fluorophores, fluorescent proteins, phosphorescent dyes, tandem dyes, particles, haptens, enzymes and radioisotopes. Techniques for binding antibodies to these labeling substances are well known in the art and can be used in the present invention. Also, in diagnostics and other assays using labeled antibodies, detection methods based on the type of label are similarly known in the art, and such detection methods can also be used in the present invention.

The antibodies of the present invention can be used for treating SARS-CoV-2 virus infection, preventing SARS-CoV-2 virus infection, and/ or detecting, diagnosing and/or prognosing SARS-CoV-2 virus infection. The antibodies of the present invention can also be used in the treatment, prevention, or diagnosis of any SARS-CoV-2 virus variant as long as they specifically bind to the antigen.

In one embodiment, the present invention is a method of treating a subject by administering to the subject an effective amount of the antibodies of the present invention. In certain embodiments, substantially purified antibodies or antigen-binding fragments thereof are used. In certain embodiments, the antibody or antigen-binding fragment thereof of the present invention is administered to a subject infected or suspected of being infected with SARS-CoV-2. In certain embodiments, the antibodies of the present invention are administered prophylactically to subjects at risk of being infected with SARS-CoV-2. In another embodiment, the antibodies of the present invention are administered to a subject after infection or after the onset of symptoms, or pre-infection prophylactically. In certain embodiments, the antibodies of the present invention are administered to infected but asymptomatic subjects.

In certain embodiments, the antibody or antigen-binding fragment thereof of the present invention, or a pharmaceutical composition containing them, and the method of the present invention of administering them to a subject treat SARS-CoV-2 viral infection or reduce viral infections in the subject. In another embodiment, the pharmaceutical compositions of the present invention and the method of the present invention prevent SARS-CoV-2 viral infection, reduce the risk of infection, or delay infection in a subject.

The subjects to which the antibodies of the present invention are administered are mammals, preferably humans. In a more particular embodiment, the subject is, for example, a subject that is immunocompromised, a subject that is particularly at risk or susceptible to infection, to the SARS-CoV-2 virus. Subjects (patients) at risk include, but not limited to, those with immunocompromised conditions, the elderly (aged 65 and older), those with underlying medical conditions such as pulmonary infections, heart disease, respiratory disease, diabetes, or dialysis patients; health care workers, persons in close contact with persons with confirmed or suspected SARS-CoV-2 infection, or persons expected to come into contact with such persons.

Administration for therapy can be a single dose schedule or a multiple dose schedule, and the antibody or antigen-binding fragment thereof of the present invention can be used in passive immunization.

In certain embodiments, the antibodies of the present invention can be administered to a subject in combination with one or more other agents, such as antiviral agents. Other agents include, but are not limited to, for example, remdesivir, favipiravir, dexamethasone, ciclenisonide, nafamostat, camostat, ivermectin, bamilanibimab, etesevimab, casilibimab, imdevimab, and HIV protease inhibitors (e.g., lopinavir, ritonavir or combination drugs thereof, and nelfinavir). In certain embodiments, the antibodies of the present invention can be administered to a subject in combination with a therapeutic agent that, if any side effects associated with the antibodies of the present invention occur, helps combat or reduce such potential side effects. Also, therapeutic agents for diseases at risk of exacerbation or causing complications in SARS-CoV-2 infection, such as diabetes, cardiovascular disease, kidney disease, and chronic respiratory diseases such as COPD can be administered in combination to subjects. Such agents include, for example, anti-inflammatory agents (e.g., corticosteroids and non-steroidal anti-inflammatory agents), anti-infective agents, nutritional supplements such as antioxidants, and any other drugs known in the art to improve the physical or mental condition of a subject.

In another embodiment, the antibodies of the present invention or the like can be administered to a subject in combination with one or more other neutralizing antibodies against SARS-CoV-2. The other antibodies may include the neutralizing antibodies mentioned above.

The present invention also provides a method for producing a pharmaceutical composition including a step of mixing the antibodies of the present invention with one or more pharmaceutically acceptable carriers.

The present invention also provides a pharmaceutical composition containing the antibodies of the present invention as active ingredients. Such pharmaceutical compositions contain a therapeutically effective amount of the antibodies of the present invention, and further contain a pharmaceutically acceptable carrier. As used herein, "pharmaceutically acceptable carrier" means a carrier approved for use by a regulatory agency or listed in a generally accepted pharmacopoeia for use in animals, particularly humans. The carriers include diluents, adjuvants, excipients, or vehicles with which the active ingredient is administered. Such carriers include sterile liquids such as water and oils such as those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The pharmaceutical composition, if desired, can also contain minor amounts of wetting or emulsifying agents, or pH buffering agents.

The pharmaceutical composition of the present invention can take any form, such as solutions, suspensions, emulsions, tablets, pills, capsules, powders, sustained-release formulations, and the like. Techniques for formulating pharmaceutical compositions into respective forms are known in the art, and can be referred to in the production of the pharmaceutical composition of the present invention, and formulations suitable for administration methods are carried out.

The dosage of the antibodies of the present invention is appropriately selected depending on the symptoms, age, condition, route of administration, and the like of the subject to be administered. When the antibody of the present invention is used for the treatment of diseases in adult patients or for the prevention of such diseases, it is usually administered, but not limited to, in a single dose at a lower limit of about 0.01 mg, preferably about 0.05 mg, and more preferably about 0.1 mg and an upper limit of about 100 mg, preferably about 50 mg, more preferably about 20 mg, and further preferably about 10 mg per kg body weight. When using an antigen-binding fragment of the antibody of the present invention, it is usual to administer a dose higher than the above dose, for example, but not limited to, about 1.5 to about 50 times, preferably about 1.5 to about 20 times, and more preferably about 1.5 to about 10 times. The frequency and interval of administration is adjusted depending on the severity of the condition. In certain embodiments, the antibodies of the present invention are administered as a starting dose of at least about 1 mg to about 1000 mg. In certain embodiments, the starting dose is followed by a second or multiple subsequent doses of an antibody or antigen-binding fragment thereof in an amount that is about the same as or less than that of the starting dose, separated by at least 1 to 3 days; at least 1 week; at least 2 weeks; at least 3 weeks; at least 4 weeks; at least 5 weeks; at least 6 weeks; at least 7 weeks; at least 8 weeks; at least 9 weeks; at least 10 weeks; or at least 12 weeks.

Administration and treatment plans, including dosage and administration schedule, can be determined appropriately by the physician who diagnosed the subject (patient), taking into consideration the symptoms, age, condition, route of administration, etc. of the subject, referring to the guidelines presented from various institutions as necessary.

The administration route of the pharmaceutical composition of the present invention can be arbitrarily determined in consideration of the symptom, condition and other conditions of the subject. Routes of administration include, but are not limited to, intradermal, transdermal, intramuscular, intraperitoneal, intravenous, subcutaneous, intranasal, epidural and oral routes. The pharmaceutical compositions are administered by any convenient route, such as by infusion or bolus injection, or by absorption through epithelial or mucocutaneous linings (e.g., oral mucosa, rectal, and intestinal mucosa, etc.), and may be administered with other biologically active agents. Administration can be systemic or local.

Various delivery systems are known for administration of active pharmaceutical ingredients, and these can be used to administer the pharmaceutical composition of the present invention. Examples thereof include encapsulation in liposomes, microparticles, nanoparticles, and microcapsules. Also, in certain embodiments, the pharmaceutical compositions can be delivered in a controlled release system. The controlled-release system places the composition near the target, thus requiring only a small systemic dose.

When administering the pharmaceutical composition of the present invention by injection, injectable preparations include intravenous, subcutaneous, intradermal, intracranial, intraperitoneal and intramuscular injection forms, or drip infusion forms and the like. These injectable preparations can be prepared with reference to known methods. Injectable preparations can be prepared, for example, by dissolving, suspending, or emulsifying the antibodies of the present invention, or a salt thereof in a sterile aqueous or oily medium commonly used for injection. Aqueous vehicles for injection include, for example, physiological saline, isotonic solutions containing glucose, and other adjuvants, and can be used in combination with a suitable solubilizer such as alcohols (e.g., ethanol), polyhydric alcohols (e.g., propylene glycol, polyethylene glycols), nonionic surfactants [e.g., polysorbate 80, HCO-50 (polyoxyethylene (50 mol) adduct of hydrogenated castor oil)], and the like. As an oily medium, for example, sesame oil, soybean oil and the like are used, and they can be used in combination with a solubilizer such as benzyl benzoate, benzyl alcohol and the like. Injections prepared are also preferably filled into suitable ampoules.

When the pharmaceutical composition of the present invention is an injection, it can be prepared as a ready-to-use injection or as a prefilled syringe. The pharmaceutical compositions of the present invention are administered subcutaneously or intravenously with standard needles and syringes. A pen delivery device can also be used for subcutaneous administration. Pen delivery devices are either reusable or disposable.

The pharmaceutical compositions, whether oral or parenteral, are prepared in dosage forms prepared in unit doses to suit the desired dosage of the active ingredient. Dosage forms containing unit doses include, for example, tablets, pills, capsules, injections (ampoules), suppositories, and the like. The amount of the antibody to be included is generally preferably from about 5 to about 1000 mg of the antibodies per dosage form in a unit dose.

The pharmaceutical compositions containing the antibodies of the present invention are useful for treating and/or preventing SARS coronavirus infection, particularly diseases, disorders, or conditions associated with SARS coronavirus such as SARS-CoV-2 infection. The pharmaceutical compositions of the present invention are also useful for ameliorating at least one symptom associated with SARS coronavirus infection, particularly SARS-CoV-2 virus infection. Such symptoms include, but are not limited to, inflammation in lungs, alveolar damage, fever, respiratory disorders (nasal congestion, runny nose, sore throat, cough, shortness of breath), headache, malaise, dysgeusia, olfactory disturbances, diarrhea, vomiting, hypercoagulability, thrombosis, Kawasaki-like vasculitis, renal dysfunction (e.g., nephritis), organ failure, pneumonia, septic shock and death. In certain embodiments, the antibodies of the present invention are useful for treating subjects suffering from severe and acute respiratory infections caused by the SARS-CoV-2 virus. In certain embodiments, the antibodies of the present invention are useful in reducing viral titers in a host. In one embodiment, the antibodies of the present invention are useful in preventing or reducing pulmonary inflammation in subjects infected with SARS-CoV-2. In one embodiment, the antibodies of the present invention are useful in preventing or reducing interstitial, peribronchiolar or perivascular inflammation, alveolar damage, and pleural changes in subjects infected with SARS-CoV-2.

The antibodies of the present invention can be used in combination with other drugs, or as a combination drug with other drugs. Alternatively, two or more of the antibodies and the like of the present invention can be used in combination, and in such a case, it is preferable to combine antibodies or antigen-binding fragments thereof that recognize different epitopes. The second therapeutic agent that can be used in combination with the antibodies of the present invention or combined with the antibodies of the present invention includes those described above. A synergistic effect is expected by these combined use or combinations.

The diagnostic method using the antibodies of the present invention includes a step of contacting a sample collected from a subject with the antibodies. Such a sample is not particularly limited as long as it is a sample that is collected from a subject and is suspected to contain a virus or part of a virus, and includes samples taken from nasal passage, sinuses, salivary gland, lung, liver, pancreas, kidney, ear, eye, placenta, gastrointestinal tract, heart, ovary, pituitary gland, adrenal gland, thyroid, brain, skin, or blood vessels (preferably peripheral vessels). Typical examples include saliva, blood, and nasal/pharyngeal swabs.

Anti-SARS-CoV-2(S) antibodies of the present invention can be used to detect or measure SARS-CoV in a sample, e.g., for diagnostic purposes. In certain embodiments, one or more antibodies of the present invention can be used in assays to detect diseases or disorders, such as viral infections. A typical diagnostic assay for SARS-CoV-2 includes, for example, a step of bringing a sample (preferably saliva, blood, or nasal/pharyngeal swab) obtained from a patient into contact with the anti-SARS-CoV-2(S) antibodies of the present invention, wherein the anti-SARS-CoV-2(S) antibody is labeled with a detectable label or reporter molecule or used as a capture ligand for selectively isolating SARS-CoV-2 from a patient sample. Alternatively, an unlabeled anti-SARS-CoV-2(S) antibody can be used in combination with a secondary antibody that itself is detectably labeled. The detectable labels or reporter molecules are radioisotopes; fluorescent or chemiluminescent moieties such as fluorescein isothiocyanate, or rhodamine; or enzymes such as alkaline phosphatase, β-galactosidase, horseradish peroxidase, or luciferase. Assay methods used to detect or measure SARS-CoV-2 in a sample include, for example, enzyme-linked immunosorbent assay (ELISA), radioimmunoassay (RIA), and fluorescence-activated cell sorting (FACS). Diagnostic and detection methods using antibodies including these are known in the art, and such known methods can be used in the present invention.

The antibodies of the present invention can also be used as a diagnostic kit for SARS-CoV-2 virus infection. Furthermore, since the antibodies of the present invention specifically bind to the RBD of the SARS-CoV-2 spike protein, it can also be used for quality control of vaccine production.

### [Examples]

The present invention will be specifically described below with reference to examples, but the present invention is not limited to the following examples.

This research project, which includes collaborators from National Hospital Organization Kyushu Medical Center and The University of Tokyo, has undergone ethical review and approval by the Factory of Life Sciences, Kumamoto University (Genome No. 461). The selection of convalescent cases who were infected with the SARS-CoV-2 Delta variant after vaccination, obtaining informed consent, and collecting blood samples were conducted by the attending physician, Dr. Yoji Nagasaki, at National Hospital Organization Kyushu Medical Center. The collected samples were then provided to the inventors of the present invention for further investigation.

### (Example 1) Identification of Cases with Cross-Neutralizing Antibodies

Blood samples from convalescent cases who had been infected with the SARS-CoV-2 Delta variant after receiving two doses of a vaccine were analyzed. A case (KUMMC) was identified in which the individual had developed potent cross-neutralizing antibodies, not only against the prototype strain (614G) but also against neutralization-resistant variants, such as the Beta variant and Mu variant.

### (Example 2) Screening of Neutralizing Monoclonal Antibodies

Peripheral blood B cells from the case identified in Example 1 were subjected to single-cell sorting using two types of spike proteins, and 532 clones producing recombinant IgG were isolated.

B cells were isolated from the plasma of two selected cases, and antibodies were recovered. Following the protocol reported by Boehmer et al. (Nature Protocols, Published online 15 September 2016; doi:10.1038/nprot.2016), peripheral blood mononuclear cells (PBMCs) were isolated from blood samples, followed by sorting using FACS Aria III to collect 7AAD-, CD19+, IgG+, IgM-, and CD27+ B cells.

After sorting, single-cell sorting was performed using two types of spike proteins (RBD [Wuhan] and RBD [SA]), and cells that bound to at least one of the spike proteins were selected. The RBD (Wuhan) and RBD (SA) proteins were provided by Professor Hashiguchi from Kyoto University. The results of the cell sorting are shown in Figure 3.

Next, following the reported by Tiller et al. (J Immunol Methods. 329: 112-124, 2008), antibodies were recovered as described below. mRNA was extracted from the obtained cells, and the variable regions of IgG were amplified by RT-PCR using the obtained mRNA. The paired genes of the heavy and light chains were selected. Each heavy and light chain gene was then cloned into an expression vector and transfected into HEK293T cells to express the antibody. The recombinant IgG (rIgG) produced in the supernatant was subsequently collected. Using this method, a total of 532 clones were recovered.

### (Example 3) Screening of SARS-CoV-2 spike protein-binding antibodies

Using a total of 532 clones obtained in Example 2, antibodies that bind to the SARS-CoV-2 spike protein were screened as described below, resulting in the identification of 176 clones.

Plasmids co-expressing the SARS-CoV-2 spike protein and EGFP were transfected into HEK293T cells to express the spike protein on the cell surface. Two days after transfection, the cells were detached with trypsin, suspended in 0.2% BSA-PBS, and used as spike protein and EGFP-expressing cells. For 50 µL of the spike protein and EGFP-expressing cell suspension, the cell culture supernatant (50 µL) of the clone obtained in Example 2 assumed to contain anti-spike antibody, or control plasma (infected plasma and normal plasma) (diluted 1: 500, 50 µL) sample was mixed and allowed to stand at room temperature for 15 minutes. The cells were separated by centrifugation, the supernatant was discarded, the cells were suspended with 0.2% BSA-PBS, washed, and then suspended with 50 µL of 200-fold diluted APC-conjugated anti-human IgG (goat antibody) antibody, and it was allowed to stand at room temperature for 15 minutes. After washing the cells with 0.2% BSA-PBS, they were fixed with 4% paraformaldehyde-PBS. The stained cells were analyzed by flow cytometry, EGFP-positive cells were gated as spike protein-expressing cells, and the spike protein-binding activity of the antibody was determined by the APC-positive rate of the EGFP⁺ cell population. As a result of flow cytometric analysis, a total of 176 clones expressing spike protein binding antibodies were screened from a total of 532 clones.

### (Example 4) Screening of antibodies that neutralize the prototype (614G) virus

Using the 176 clones obtained in Example 3, antibodies capable of neutralizing the mutant virus with the D614G spike mutation (prototype (614G) virus) were screened as described below, referring to Kaku et al. (Cell Rep. 2021 Jul 13; 36(2):109385), resulting in that 18 clones were identified.

Plasmids co-expressing antibody heavy and light chains were transfected into HEK293T cells, and the SARS-CoV-2 neutralizing activity of the recombinant antibody contained in the cell culture supernatant was examined in a pseudovirus neutralization test. Pseudoviruses were made by transfecting into HEK293T cells with packaging plasmid psPAX2-IN/HiBiT, transfer plasmid pWPI-Luc2, and SARS-CoV-2 Spike expression plasmid pCMV3-SARS2-S at a ratio of 2: 2: 1. The SARS-CoV-2 Spike expression plasmid was produced by modifying the wild-type plasmid obtained from Sino Biological Inc. and introducing the prototype (614G) mutation.

Two days after transfection, the culture supernatant was collected, passed through a 0.22 µm filter, stored at - 80°C, and used for the following tests. For the neutralization test, 293FT-DSP1-7-ACE2-TMPRSS2 cells (provided by Professor Inoue, The Institute of Medical Science, The University of Tokyo) were used, and the day before, 1×10⁵ cells/mL cell suspension was seeded at 100 µL/well in a 96-well plate. To another 96-well plate, the cell culture supernatant of each clone containing the recombinant antibody was diluted twice with culture medium and added at 110 µL/well, and then 200 TCID₅₀ of pseudovirus was added at 110 µL/well and cultured at 37°C for 1 hour (hereinafter referred to as "mixture of culture supernatant and virus"). The experiment was performed with 2 wells for each sample. The cell culture supernatant prepared the day before was discarded, and a mixture of the culture supernatant and virus was added and cultured. After 5 hours, the mixture of the culture supernatant and virus was discarded, 200 µL of fresh culture medium was added, and the cells were further cultured. Two days after infection, the cell supernatant was discarded, 30 µL of lysis buffer was added, and the cells were lysed by stirring for 10 minutes. 10 µL of the cell lysate was transferred to a white 96-well plate, 50 µL of luciferase substrate was added, and fluorescence was measured. The RLU (relative light unit) of the virus-only sample was defined as 100% infection, and the RLU of the virus-free sample was defined as 0% infection, and the neutralizing activity by the cell culture supernatant of each clone was determined by how much RLU was reduced from 100% infection. The neutralizing activity of the cell culture supernatant of the clones that showed SARS-CoV-2 spike protein-binding activity were measured. Two independent assays were conducted, and clones that exhibited neutralizing activity of approximately 40% or higher in both assays were selected as neutralizing antibodies. As a result, 18 clones were selected.

### (Example 5) Screening of Antibodies That Neutralize the Omicron Variant

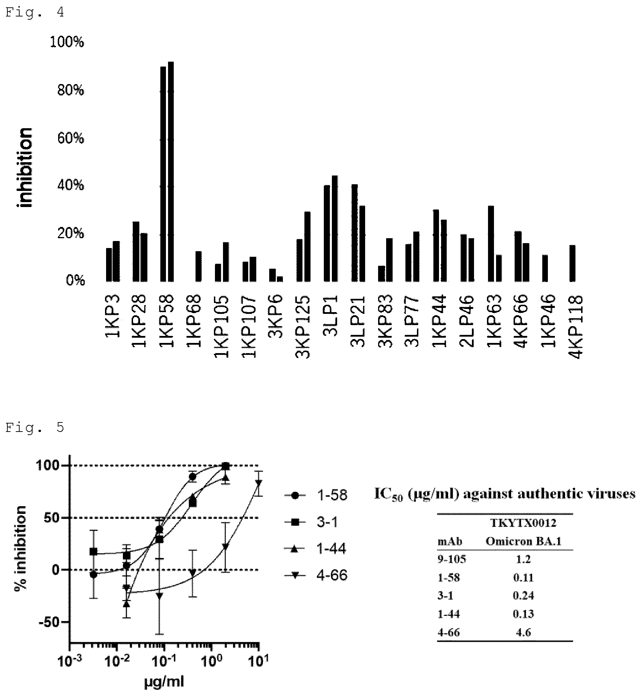

Using the 18 clones obtained in Example 4, antibodies capable of neutralizing the Omicron variant were screened. Specifically, the wild-type spike protein plasmid obtained from Sino Biological Inc. was modified to generate a spike protein carrying the mutations of the Omicron BA.1 variant. The screening was then conducted in the same manner as in Example 4. The results of the neutralization activity assay are shown in Figure 4.

Based on the neutralization activity results, clones 1-58, 3-1, 3-21, 1-44, and 4-66 were selected. Amino acid sequence analysis revealed that clones 3-1 and 3-21 had identical amino acid sequences.

The heavy chain variable region and light chain variable region amino acid sequences of the four identified clones (1-58, 3-1, 1-44, and 4-66) are shown in Figure 1.

Analysis of the heavy chain variable regions and light chain variable regions of the four selected clones revealed that, as shown in Figure 5, 1-58 antibody has the heavy chain derived from IGHV1-46 and the light chain derived from IGKV1-39, 3-1 antibody has the heavy chain derived from IGHV5-51 and the light chain derived from IGLV1-44, 1-44 antibody has the heavy chain derived from IGHV3-33 and the light chain derived from IGKV1-39, and 4-66 antibody has the heavy chain derived from IGHV3-53 and the light chain derived from IGKV1-33.

An overview of the genetic characteristics of the heavy and light chain variable regions of the four selected clones is summarized in Table 2.

**Table 2:**

| **Genetic summary of mabs 1-58, 3-1 and 4-66** | | | | |
|---|---|---|---|---|
| sequence | V gene | V gene identity (%) | CDR3 amino acids | CDR3 length |
| 1-58H | IGHV1-46 | 94.218 | TRSTRDYVWGNYRYTPGYYLDY | 22 |
| 1-58K | IGKV1-39 | 96.831 | QQGYITPIT | 9 |
| 3-1H | IGHV5-51 | 97.297 | ARQQGGFGDLGAYNWFDP | 18 |
| 3-1L | IGLV1-44 | 96.939 | AAWDDSLNGWV | 11 |
| 1-44H | IGHV3-33 | 96.622 | AREGIAVPGNGADAFDI | 17 |
| 1-44L | IGKV1-39 | 97.535 | QQTYSTPYT | 9 |
| 4-66H | IGHV3-53 | 95.563 | ARDPPHRRGSY | 11 |
| 4-66L | IGKV1-33 | 97.561 | QQSDNLPPT | 9 |

Additionally, the 9-105 antibody, previously reported by the inventors, is derived from IGHV3-53 and IGKV3-20. LY-CoV1404 and Sotrovimab, which are previously reported, is derived from IGHV2-5 and IGLV2-14, and IGHV1-18 and IGKV3-20, respectively. For further information on approved antibodies, refer to Int. J. Mol. Sci. 2022, 23, 9763 [DOI: 10.3390/ijms23179763].

### (Example 6) Neutralization assay of the Omicron variant using purified antibodies

The antibody genes of the selected four clones were transfected and expressed in CHO cells, and purified antibodies were prepared. The purified antibodies were subjected to a neutralization assay. The transfection of antibody genes into CHO cells and the preparation of purified antibodies from the culture supernatant were performed using the ExpiCHO Expression System (Thermo Fisher) following standard protocols. The neutralizing activity was confirmed using a plaque reduction assay with live Omicron BA.1 virus (authentic Omicron variant). Specifically, the assay was performed as described below, referring to Kaku et al., Cell Rep. 2021 Jul 13; 36(2):109385.

Diluted antibody (110 µL) was mixed with 110 PFU (plaque-forming units) of the Omicron BA.1 (TKYTX0012) strain and incubated at 37°C for 1 hour. The antibody-virus mixture (40 µL and 160 µL) was added to Vero E6 cells seeded the previous day in a 12-well plate at 2×10⁵ cells/well. After 2 hours of incubation, 1 mL of PFU buffer (1× MEM, 3% FBS, 1.5% carboxymethylcellulose) was overlaid. The cells were incubated for 3 days, washed three times with PBS, and fixed with 4% paraformaldehyde-PBS. After washing with running water and drying, plaques were stained with 0.1% methylene blue and counted. The neutralization activity data and the calculated IC₅₀ values were summarized in Figure 5.

The antibodies 1-58, 3-1, 1-44, and 4-66 neutralized the Omicron BA.1 virus with IC₅₀ values ranging from 0.011 to 4.6 µg/mL.

### (Example 7) Neutralization assay using various SARS-CoV-2 variants

A neutralization assay of the four selected antibodies (1-58, 3-1, 1-44, and 4-66 antibodies) against various known SARS-CoV-2 variants was conducted. Specifically, it was evaluated by a pseudovirus-based neutralization assay using various variants, as described below.

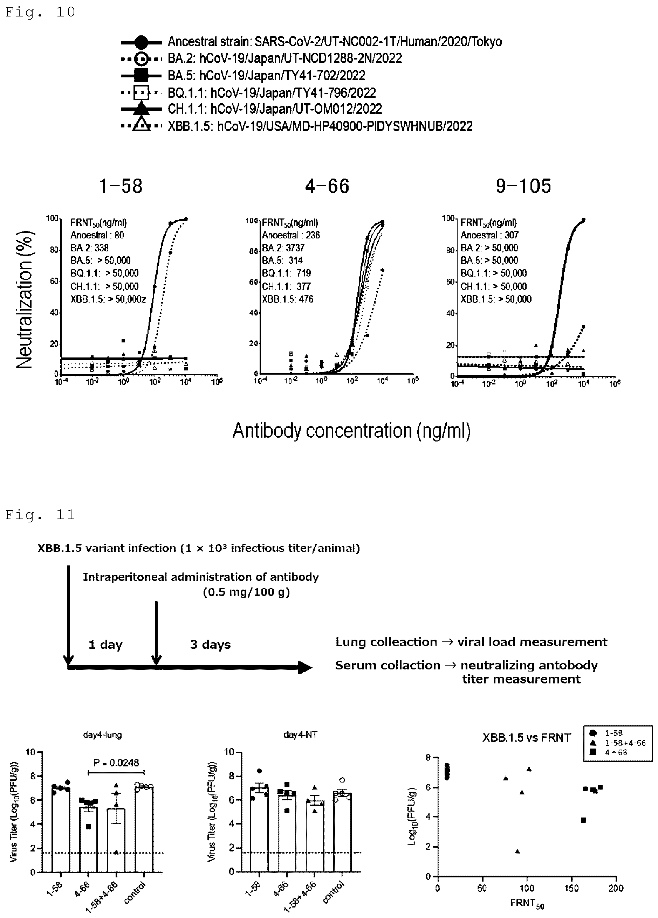

Expression plasmids encoding the spike proteins of SARS-CoV-2 variants (614G, Alpha, Beta, Gamma, Delta, Kappa, Lambda, Mu, BA.1, BA.2, BA.3, BA.1.1, BA.2.12.1, and BA.4/5) were generated by introducing mutations into the wild-type spike expression plasmid via PCR-based mutagenesis. FIG. 11 shows the amino acid mutations in the Spike protein of each mutant strain. The spike expression plasmid of the SARS-CoV-2 mutant strain, and the lentiviral packaging plasmid psPAX2-IN/HiBiT, transfer plasmid pWPI-Luc2 were together transfected into 293 T cells, and the supernatant after two days was harvested and frozen, and used as pseudoviruses.

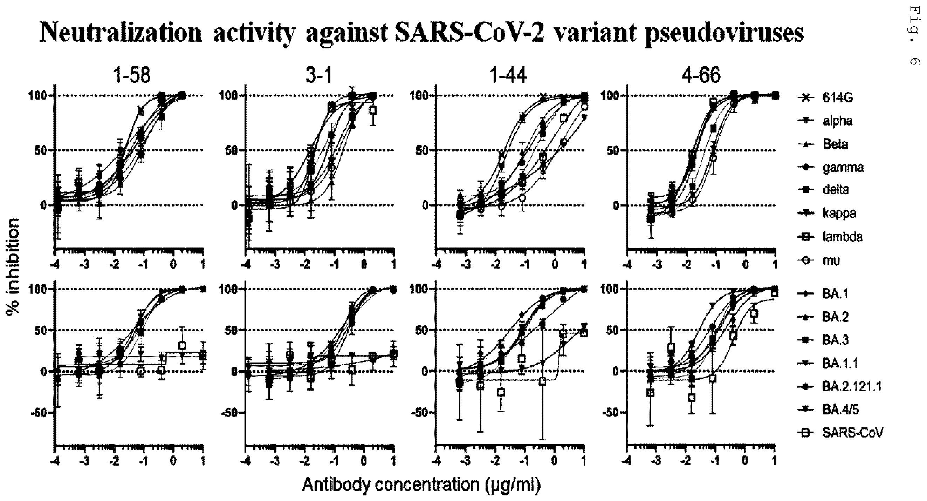

Four monoclonal antibodies screened were used to measure neutralizing activity. 200 TCID₅₀ (100 µL) of the pseudovirus was added to the diluted antibody (100 µL), incubated for 1 hour, and added to 293FT/DSP/ACE2/TMPRSS2 cells in a 96 well plate. After 2 hours of incubation, the mixed solution of antibody and virus was discarded and fresh medium was added. After 48 hours of incubation, luciferase activity was measured with a luciferase assay system (Promega) and an EnSpire Multimode Plate Reader (PerkinElmer). The measured neutralization data are shown in Figure 6.

1-58 antibody neutralized all variants with IC₅₀ < 0.087 µg/mL. 3-1 antibody showed some variability but generally neutralized variants with IC₅₀ < 0.25 µg/mL.1-44 antibody required higher concentrations to neutralize Delta, Mu, and Omicron BA.4/5 variants, but neutralized other variants with IC₅₀ < 0.47 µg/mL. 4-66 antibody neutralized all variants with IC₅₀ < 0.21 µg/mL and also neutralized SARS-CoV with IC₅₀ = 0.55 µg/mL. These results indicate that the antibodies of the present invention effectively inhibit most variants at IC₉₀ = 1 µg/mL. Given that the Cₘₐₓ of a monoclonal antibody after intravenous administration at 1 mg/kg is estimated to be 20-30 µg/mL, it is expected that a dosage of 50-100 mg of the antibodies of the present invention would be sufficient to achieve a therapeutic effect for all infection cases.

### (Example 8) RBD-ACE2 binding inhibition assay

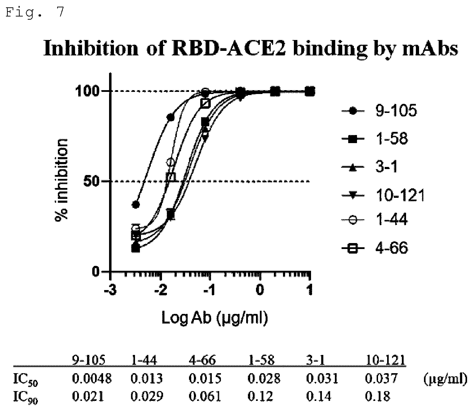

Using the Cayman Chemical kit (#502050), the RBD-ACE2 inhibition effect was examined according to the manufacturer's protocol. In addition to the four antibodies (1-58, 3-1, 1-44, and 4-66) of the present invention, the antibodies (9-105 and 10-121), which the inventors previously reported, were also tested for their inhibitory activity. The measured inhibition activity data and the calculated IC₅₀ results are shown in Figure 7.

1-58 and 3-1 exhibited inhibitory activity comparable to 10-121, while 1-44 and 4-66 exhibited inhibition, approximately midway between 9-105 and 10-121.

### (Example 9) Comparative study with existing antibodies

A comparative study of neutralizing activity against various variants was conducted in the same manner as in Example 7, comparing with existing antibodies. REGN-10933 and REGN-10987 (Regeneron/Roche), LY-CoV555 (Eli Lilly), and VIR-7831 and VIR-7832 (GSK) used were as the existing antibodies. These existing antibodies were produced using a CHO cell expression system, based on publicly known information about these antibodies. Additionally, previously reported antibodies by the inventors, 9-105 and 10-121, were also included in the comparison.

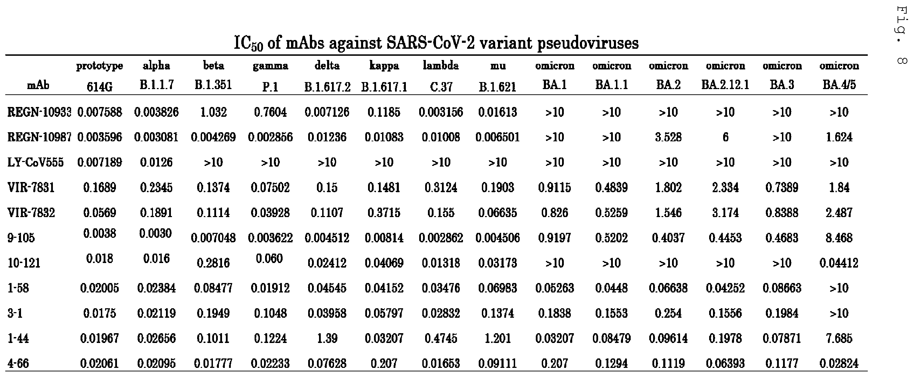

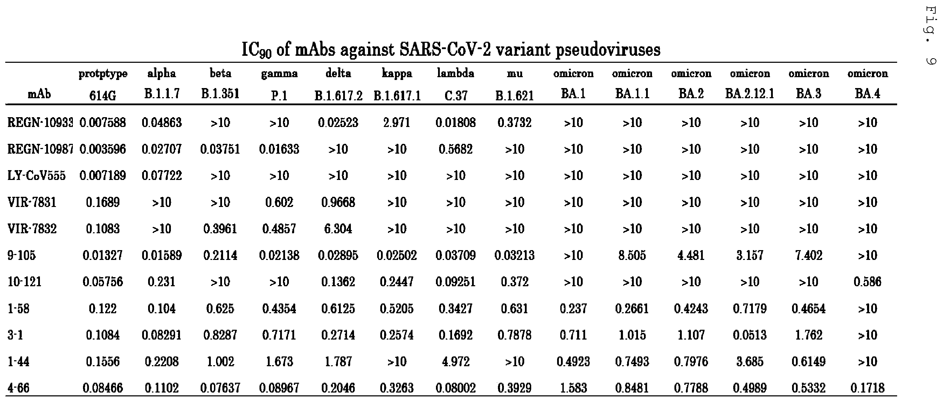

The IC₅₀ and IC₉₀ values of the antibodies against various variants are shown in Figures 8 and 9.

The potent neutralizing antibody (9-105 antibody) previously reported by the inventors and the approved coronavirus therapeutic antibody REGN-10987 are effective at low concentrations against existing viruses, including 614G, but have reduced efficacy against the Omicron variant. Additionally, the 9-105 antibody exhibits neutralizing activity against the Omicron variant at a titer comparable to Sotrovimab (VIR-7831), but requires a concentration more than 50 times higher compared to its neutralizing activity against other viral variants. On the other hand, the antibodies of the present invention (1-58, 3-1, 1-44, and 4-66) are effective against most variants, including the Omicron variant and demonstrated better neutralizing activity at lower concentrations against the Omicron variant compared to 9-105 antibody and Sotrovimab.

The potent neutralizing antibodies (9-105 antibody and 10-121 antibody) previously reported by the inventors and the neutralizing antibodies of the present invention exhibit different spectrums against various SARS-CoV-2 variants. Therefore, by combining the 9-105 and 10-121 antibodies with the antibodies of the present invention (1-58, 3-1, 1-44, and 4-66), it is expected to serve as a potent preventive and therapeutic agent against SARS-CoV-2. Similarly, the antibodies of the present invention can also be used in combination with existing neutralizing antibodies.

### (Example 10) Neutralization assay against various Omicron variants

An assay was conducted to evaluate the neutralizing activity of two antibodies (1-58 and 4-66) against various Omicron subvariants (BA.2, BA.5, BQ.1.1, CH.1.1, and XBB.1.5). As a control, the 9-105 antibody, which was previously reported by the inventors, was used. Specifically, the neutralizing activity was measured as follows.

Monoclonal antibodies were serially diluted fivefold in PBS in a 96-well plate (maximum concentration: 50,000 ng/mL) and mixed with 100-400 FFU (focus-forming units) of the virus per well, then incubated at 37°C for 1 hour. The serum-virus mixture was inoculated into Vero E6-TMPRSS2-T2A-ACE2 cells in a 96-well plate (two wells per sample). After incubation at 37°C for 1 hour, 100 µL of 1.5% methylcellulose 400 (FUJIFILM Wako Pure Chemical Corporation) dissolved in medium was added to each well. The cells were incubated at 37°C for 14-18 hours and fixed with formalin. After removing the formalin, the cells were immunostained with a mouse monoclonal antibody against the SARS-CoV-2 nucleocapsid (N) protein [N45 (TAUNS Laboratories, Inc., Japan)], followed by immunostaining with an HRP-labeled goat anti-mouse immunoglobulin antibody (Jackson ImmunoResearch). The fixed infected cells were stained with TrueBlue Substrate (SeraCare Life Sciences) and washed with distilled water. After drying the cells, the Focus count was quantified using the ImmunoSpot S6 Analyzer, ImmunoCapture Software, and BioSpot Software (Cellular Technology). The results were calculated as the 50% Focus Reduction Neutralization Titer (FRNT₅₀) using GraphPad Prism (GraphPad Software).

The results are shown in Figure 10. While the 9-105 antibody exhibited neutralizing activity only against the ancestral (wildtype) strain, the 1-58 antibody also demonstrated neutralizing activity against the Omicron BA.2 variant. Surprisingly, the 4-66 antibody exhibited sufficient neutralizing activity against all variants.

### (Example 11) Therapeutic effect of antibodies in a coronavirus-infected animal model

The therapeutic effect of the antibodies of the present invention (1-58 and 4-66) was evaluated in a hamster model infected with an Omicron lineage variant, as described below. Six-week-old male Syrian hamsters (Japan SLC) were used. To assess the efficacy of monoclonal antibodies in hamsters, five hamsters per group were anesthetized with isoflurane and intranasally inoculated with 103 PFU (Plaque-Forming Units) of the Omicron lineage XBB.1.5 variant (hCoV-19/USA/MD-HP40900-PIDYSWHNUB/2022) in 30 µL. On Day 1 post-infection, 1 mL of monoclonal antibody (5 mg/kg) diluted in PBS was intraperitoneally injected into the hamsters. On Day 4 post-infection, the animals were euthanized, and viral titers in the nasal turbinates and lungs were measured using a plaque assay with Vero E6/TMPRSS2 cells. Serum antibody titers were measured using hamster serum as described below.

Hamster serum was first treated at 56°C for 1 hour. The treated serum samples were then serially diluted fivefold in DMEM containing 2% FCS (fetal calf serum) in a 96-well plate and mixed with 100-400 FFU (focus-forming units) of the virus per well, followed by incubation at 37°C for 1 hour. The serum-virus mixture was then inoculated into Vero E6-TMPRSS2-T2A-ACE2 cells in a 96-well plate (two wells per sample). After incubation at 37°C for 1 hour, 100 µL of 1.5% methylcellulose 400 (FUJIFILM Wako Pure Chemical Corporation) dissolved in medium was added to each well. The cells were incubated at 37°C for 14-18 hours and then fixed with formalin. After removing formalin, the cells were immunostained using a rabbit monoclonal antibody against the SARS-CoV-2 nucleocapsid (N) protein (Sino Biological Inc.) and subsequently stained with an HRP-labeled goat anti-rabbit immunoglobulin antibody (Jackson ImmunoResearch). The fixed infected cells were stained using TrueBlue Substrate (SeraCare Life Sciences) and washed with distilled water. After drying the cells, the Focus count was quantified using the ImmunoSpot S6 Analyzer, ImmunoCapture Software, and BioSpot Software (Cellular Technology). The results were calculated as the 50% Focus Reduction Neutralization Titer (FRNT₅₀) using GraphPad Prism (GraphPad Software).

The experimental overview is shown in the upper panel of Figure 11, while the experimental results are shown in the lower panel of Figure 11. Administration of the 4-66 antibody led to a significant reduction in viral load in the lungs. These findings demonstrate that the 4-66 antibody exhibits in vivo therapeutic efficacy against the novel Omicron subvariant XBB.1.5.

The above detailed description merely illustrates objects and subjects of the present invention and is not intended to limit the accompanying Claims. Without departing from the accompanying Claims, various modifications and alterations to the embodiments described will be apparent to those skilled in the art in view of the teachings herein.

This application claims priority to Japanese Patent Application No. 2022-142801 (filed on September 8, 2022), the entire contents of which are incorporated herein by reference.

### [Industrial Applicability]

The present invention provides antibodies against SARS-CoV-2 variants. The antibodies of the present invention exhibit neutralizing activity against variants such as the Omicron variant and are useful as therapeutic agents for coronavirus infections.

## Claims

1. An antibody or antigen binding fragment thereof that binds to the spike protein of the SARS-CoV-2 and has the ability to neutralize the SARS-CoV-2 virus (particularly the Omicron variant), comprising the following heavy chains CDR1-3 and light chains CDR1-3:
(1) a heavy chain CDR1 selected from the group consisting of:
a heavy chain CDR1 represented by SEQ ID NO: 9 (GYSFTASY),
a heavy chain CDR1 represented by SEQ ID NO: 11 (GYIFTNYW),
a heavy chain CDR1 represented by SEQ ID NO: 13 (GFTFRSHA),
a heavy chain CDR1 represented by SEQ ID NO: 15 (GIIVSRNY),
a heavy chain CDR1 consisting of a sequence in which one amino acid is deleted, substituted or added in any of heavy chains CDR1 selected from the group consisting of the sequences represented by SEQ ID NOs: 9, 11, 13 and 15, and
a heavy chain CDR1 having 85% or more substantial identity to any of heavy chains CDR1 selected from the group consisting of the sequences represented by SEQ ID NOs: 9, 11, 13 and 15,
(2) a heavy chain CDR2 selected from the group consisting of:
a heavy chain CDR2 represented by SEQ ID NO: 17 (INPGDDNT),
a heavy chain CDR2 represented by SEQ ID NO: 18 (INPRDSDT),
a heavy chain CDR2 represented by SEQ ID NO: 19 (IWYDGSNK),
a heavy chain CDR2 represented by SEQ ID NO: 20 (IYSGGTT),
a heavy chain CDR2 consisting of a sequence in which one amino acid is deleted, substituted or added in any of heavy chains CDR2 selected from the group consisting of the sequences represented by SEQ ID NOs: 17, 18, 19 and 20, and
a heavy chain CDR2 having 85% or more substantial identity to any of heavy chains CDR2 selected from the group consisting of the sequences represented by SEQ ID NOs: 17, 18, 19 and 20,
(3) a heavy chain CDR3 selected from the group consisting of:
a heavy chain CDR3 represented by SEQ ID NO: 21 (TRSTRDYVWGNYRYTPGYYLDY),
a heavy chain CDR3 represented by SEQ ID NO: 23 (ARQQGGFGDLGAYNWFDP),
a heavy chain CDR3 represented by SEQ ID NO: 25 (AREGIAVPGNGADAFDI),
a heavy chain CDR3 represented by SEQ ID NO: 27 (ARDPPHRRGSY),
a heavy chain CDR3 consisting of a sequence in which one or
two amino acids are deleted, substituted or added in any of heavy chains CDR3 selected from the group consisting of the sequences represented by SEQ ID NOs: 21, 23, 25 and 27, and
a heavy chain CDR3 having 90% or more substantial identity to any of heavy chains CDR3 selected from the group consisting of the sequences represented by SEQ ID NOs: 21, 23, 25 and 27,
(4) a light chain CDR1 selected from the group consisting of:
a light chain CDR1 represented by SEQ ID NO: 10 (QTISNY),
a light chain CDR1 represented by SEQ ID NO: 12 (SSNIGSNT),
a light chain CDR1 represented by SEQ ID NO: 14 (QSIGNF),
a light chain CDR1 represented by SEQ ID NO: 16 (QDINKY),
a light chain CDR1 consisting of a sequence in which one amino acid is deleted, substituted or added in any of light chains CDR1 selected from the group consisting of the sequences represented by SEQ ID NOs: 10, 12, 14 and 16, and
a light chain CDR1 having 80% or more substantial identity to any of light chains CDR1 selected from the group consisting of the sequences represented by SEQ ID NOs: 10, 12, 14 and 16,
(5) a light chain CDR2 selected from the group consisting of:
a light chain CDR2 represented by the sequence A (AAS),
a light chain CDR2 represented by the sequence B (RDH), and
a light chain CDR2 represented by the sequence C (DAS), and (6) a light chain CDR3 selected from the group consisting of:
a light chain CDR3 represented by SEQ ID NO: 22(QQGYITPIT),
a light chain CDR3 represented by SEQ ID NO: 24 (AAWDDSLNGWV),
a light chain CDR3 represented by SEQ ID NO: 26(QQTYSTPYT),
a light chain CDR3 represented by SEQ ID NO: 28(QQSDNLPPT),
a light chain CDR3 consisting of a sequence in which one amino acid is deleted, substituted or added in any of light chains CDR3 selected from the group consisting of the sequences represented by SEQ ID NOs: 22, 24, 26 and 28, and
a light chain CDR3 having 85% or more substantial identity with any of light chains CDR3 selected from the group consisting of the sequences represented by SEQ ID NOs: 22, 24, 26 and 28.

2. The antibody or antigen-binding fragment thereof according to claim 1, wherein the heavy chain CDR1 is a heavy chain CDR1 selected from the group consisting of the sequences represented by SEQ ID NOs: 9, 11, 13 and 15, the heavy chain CDR2 is a heavy chain CDR2 selected from the group consisting of the sequences represented by SEQ ID NOs: 17, 18, 19 and 20, the heavy chain CDR3 is a heavy chain CDR3 selected from the group consisting of the sequences represented by SEQ ID NOs: 21, 23, 25 and 27, the light chain CDR1 is a light chain CDR1 selected from the group consisting of the sequences represented by SEQ ID NOs: 10, 12, 14 and 16, the light chain CDR2 is a light chain CDR2 selected from the group consisting of the sequences A, B and C, and the light chain CDR3 is a light chain CDR3 selected from the group consisting of the sequences represented by SEQ ID NOs: 22, 24, 26 and 28.

3. The antibody or antigen-binding fragment thereof according to claim 1, wherein the combination of heavy chain CDR1, heavy chain CDR2, heavy chain CDR3, light chain CDR1, light chain CDR2, and light chain CDR3 is selected from the group consisting of:
(1) the heavy chain CDR1 represented by SEQ ID NO: 9, the heavy chain CDR2 represented by SEQ ID NO: 17, the heavy chain CDR3 represented by SEQ ID NO: 21, the light chain CDR1 represented by SEQ ID NO: 10, the light chain CDR2 represented by the sequence A, and the light chain CDR3 represented by SEQ ID NO: 22.
(2) the heavy chain CDR1 represented by SEQ ID NO: 11, the heavy chain CDR2 represented by SEQ ID NO: 18, the heavy chain CDR3 represented by SEQ ID NO: 23, the light chain CDR1 represented by SEQ ID NO: 12, the light chain CDR2 represented by the sequence B, and the light chain CDR3 represented by SEQ ID NO: 24.
(3) the heavy chain CDR1 represented by SEQ ID NO: 13, the heavy chain CDR2 represented by SEQ ID NO: 19, the heavy chain CDR3 represented by SEQ ID NO: 25, the light chain CDR1 represented by SEQ ID NO: 14, the light chain CDR2 represented by the sequence A, and the light chain CDR3 represented by SEQ ID NO: 26.
(4) the heavy chain CDR1 represented by SEQ ID NO: 15, the heavy chain CDR2 represented by SEQ ID NO: 20, the heavy chain CDR3 represented by SEQ ID NO: 27, the light chain CDR1 represented by SEQ ID NO: 16, the light chain CDR2 represented by the sequence C, and the light chain CDR3 represented by SEQ ID NO: 28.

4. The antibody or antigen-binding fragment thereof according to claim 1, comprising the heavy chain variable region represented by SEQ ID NO: 1 and the light chain variable region represented by SEQ ID NO: 2.

5. The antibody or antigen-binding fragment thereof according to claim 1, comprising the heavy chain variable region represented by SEQ ID NO: 3 and the light chain variable region represented by SEQ ID NO: 4.

6. The antibody or antigen-binding fragment thereof according to claim 1, comprising the heavy chain variable region represented by SEQ ID NO: 5 and the light chain variable region represented by SEQ ID NO: 6.

7. The antibody or antigen-binding fragment thereof according to claim 1, comprising the heavy chain variable region represented by SEQ ID NO: 7 and the light chain variable region represented by SEQ ID NO: 8.

8. The antibody or antigen-binding fragment thereof according to any one of claims 1 to 7, wherein in a neutralization assay using pseudoviruses, the antibody activity in neutralizing the BA.1 strain of the SARS-CoV-2 Omicron variants has a neutralizing ability expressed as 50% inhibitory concentration (IC₅₀ µg/mL) within the range of about 1 µg/mL or less (preferably about 0.8 µg/mL or less, more preferably about 0.5 µg/mL or less).

9. The antibody or antigen-binding fragment thereof according to any one of claims 1 to 7, having neutralizing activity against at least four (preferably at least five) variants selected from the group consisting of the SARS-CoV-2 Omicron variants BA.1, BA.1.1, BA.2, BA.2.12.1, BA.3, BA.4/5, BQ.1.1, CH.1.1, and XBB.1.5, as expressed as 50% inhibitory concentration (IC₅₀ µg/mL) within the range of about 1 µg/mL or less (preferably about 0.8 µg/mL or less, more preferably about 0.5 µg/mL or less, and even more preferably about 0.2 µg/mL or less).

10. The antibody or antigen-binding fragment thereof according to any one of claims 1 to 9, selected from the group consisting of immunoglobulin molecules, monoclonal antibodies, human antibodies, chimeric antibodies, CDR-grafted antibodies, Fab, Fab', F(ab')2, Fd, Fv, disulfide-bound Fv, scFv, single domain antibodies, nanobody antibodies, diabody antibodies, bispecific antibodies, and multi-specific antibodies.

11. A nucleic acid encoding the antibody or antigen-binding fragment thereof according to any one of claims 1 - 10.

12. A vector comprising the nucleic acid according to claim 11.

13. A host cell comprising the vector according to claim 12.

14. A method for producing the antibody or antigen-binding fragment thereof according to any one of claims 1 to 7, comprising a step of culturing the host cell under conditions suitable for expressing the antibody or antigen-binding fragment thereof.

15. A pharmaceutical composition comprising the antibody or antigen-binding fragment thereof according to any one of claims 1 to 7 and a pharmaceutically acceptable carrier.

16. The pharmaceutical composition according to claim 15, further comprising at least one additional neutralizing antibody or its antigen-binding fragment thereof against SARS-CoV-2 and/or SARS-CoV-2 variants.

17. The pharmaceutical composition according to claim 16, wherein the additional neutralizing antibody or its antigen-binding fragment thereof is an antibody or its antigen-binding fragment thereof comprising the heavy chain CDR1 represented by SEQ ID NO: 33 (GITVSSNY), the heavy chain CDR2 represented by SEQ ID NO: 37 (IYSGGST), the heavy chain CDR3 represented by SEQ ID NO: 38 (ARDLEAAGGMDV), the light chain CDR1 represented by SEQ ID NO: 34 (QSVPSIY), the light chain CDR2 represented by the sequence D (GAS) and the light chain CDR3 represented by SEQ ID NO: 39 (QQYGSSPGT), or an antibody or its antigen-binding fragment thereof comprising the heavy chain CDR1 represented by SEQ ID NO: 35 (GLTVSRNY), the heavy chain CDR2 represented by SEQ ID NO: 37 (IYSGGST), the heavy chain CDR3 represented by SEQ ID NO: 40 (ARPIVGARAGMDV), the light chain CDR1 represented by SEQ ID NO: 36 (QDISNY), the light chain CDR2 represented by the sequence E (DAS) and the light chain CDR3 represented by SEQ ID NO: 41 (QQYDNLPGT).

18. The pharmaceutical composition according to claim 16, wherein the additional neutralizing antibody or its antigen-binding fragment thereof is an antibody or its antigen-binding fragment thereof comprising the heavy chain variable region represented by SEQ ID NO: 29 and the light chain variable region represented by SEQ ID NO: 30, or an antibody or its antigen-binding fragment thereof comprising the heavy chain variable region represented by SEQ ID NO: 31 and the light chain variable region represented by SEQ ID NO: 32.

19. A pharmaceutical composition for the prevention or treatment of SARS-CoV-2 virus infection according to claim 15.

20. The pharmaceutical composition according to claim 19, further comprising at least one additional neutralizing antibody or its antigen-binding fragment thereof against SARS-CoV-2 and/or SARS-CoV-2 variants.

21. The pharmaceutical composition according to claim 20, wherein the additional neutralizing antibody or its antigen-binding fragment thereof is an antibody or its antigen-binding fragment thereof comprising the heavy chain CDR1 represented by SEQ ID NO: 33 (GITVSSNY), the heavy chain CDR2 represented by SEQ ID NO: 37 (IYSGGST), the heavy chain CDR3 represented by SEQ ID NO: 38 (ARDLEAAGGMDV), the light chain CDR1 represented by SEQ ID NO: 34 (QSVPSIY), the light chain CDR2 represented by the sequence D (GAS) and the light chain CDR3 represented by SEQ ID NO: 39 (QQYGSSPGT), or an antibody or its antigen-binding fragment thereof comprising the heavy chain CDR1 represented by SEQ ID NO: 35 (GLTVSRNY), the heavy chain CDR2 represented by SEQ ID NO: 37 (IYSGGST), the heavy chain CDR3 represented by SEQ ID NO: 40 (ARPIVGARAGMDV), the light chain CDR1 represented by SEQ ID NO: 36 (QDISNY), the light chain CDR2 represented by the sequence E (DAS) and the light chain CDR3 represented by SEQ ID NO: 41 (QQYDNLPGT).

22. The pharmaceutical composition according to claim 20, wherein the additional neutralizing antibody or its antigen-binding fragment thereof is an antibody or its antigen-binding fragment thereof comprising the heavy chain variable region represented by SEQ ID NO: 29 and the light chain variable region represented by SEQ ID NO: 30, or an antibody or its antigen-binding fragment thereof comprising an antibody or its antigen-binding fragment thereof comprising the heavy chain variable region represented by SEQ ID NO: 31 and the light chain variable region represented by SEQ ID NO: 32.

23. The pharmaceutical composition according to claim 19, **characterized by** being administered in combination with other anti-coronaviral drug(s).

24. The pharmaceutical composition according to claim 23, wherein the other anti-coronaviral drug(s) is at least one anti-coronaviral drug selected from remdesivir, favipiravir, dexamethasone, ciclenisonide, nafamostat, camostat, ivermectin, bamilanibimab, etesevimab, casilibimab, imdevimab, and HIV protease inhibitors (for example, Lopinavir, ritonavir or their combination, nelfinavir).

25. Use of the antibody or its antigen-binding fragment thereof according to any one of claims 1 to 7 in the manufacture of a pharmaceutical composition for the prevention or treatment of SARS-CoV-2 virus infection.
